# EUROPEAN PATENT APPLICATION

(11) **EP 1 719 515 A2**
(43) Date of publication of application: **08.11.2006**
(21) Application number: 06010935.2
(22) Date of filing: 07.06.2004
(51) Int. Cl.: A61K 31/704, A61K 31/7042, A61P 31/12

(54) **Glycyrrhizin or derivatives thereof for treating or preventing togavirus infections**

(30) Priority: 06.06.2003 US 476908 P
(62) Divisional of application: 04744051.6
(71) Applicant: Johann Wolfgang Goethe University, 60325 Frankfurt am Main (DE)
(72) Inventor: Cinatl, Jindrich, 63069 Offenbach a.M. (DE); Doerr, Hans Wilhelm, 63303 Dreieich (DE); Hover, Gerold, 63067 Offenbach a.M. (DE); Michaelis, Martin, 60439 Frankfurt a.M. (DE)
(74) Representative: Sonn & Partner Patentanwälte

(57) **Abstract**

The invention provides means for preventing, treating, managing or ameliorating viral infections, in particular togavirus. More specifically, the invention provides the use of Glycyrrhizin and/or derivatives thereof for the preparation of a medicament for preventing, treating, managing or ameliorating a togavirus or one or more symptoms thereof. The invention also provides the use of Glycyrrhizin and/or a derivative thereof in combination with a prophylactic or therapeutic agent other than Glycyrrhizin or a derivative thereof for the preparation of a medicament for preventing, treating, managing or ameliorating a togavirus or one or more symptoms thereof.

## Description

### RELATED APPLICATIONS

This application claims the benefit of U.S. Provisional Application no. 60/476,908 filed June 6, 2003, the entire disclosure of which is incorporated herein by reference in its entirety.

### 1. INTRODUCTION

The invention provides methods for preventing, treating, managing or ameliorating viral infections, in particular Severe Acute Respiratory Syndrome (SARS). More specifically, the invention provides methods for preventing, treating, managing or ameliorating a SARS-associated coronavirus or one or more symptoms thereof by administering Glycyrrhizin and/or derivatives thereof. The invention also provides methods for preventing, treating, managing or ameliorating a SARS-associated coronavirus or one or more symptoms thereof by administering Glycyrrhizin and/or a derivative thereof in combination with a prophylactic or therapeutic agent other than Glycyrrhizin or a derivative thereof.

### 2. BACKGROUND OF THE INVENTION

### 2.1. SEVERE ACUTE RESPIRATORY SYNDROME (SARS)

A new coronavirus has been found in patients with Severe Acute Respiratory Syndrome (SARS) and has been identified as the probable cause of SARS (SARS; Drosten et al., 2003, N Engl J Med 348:1967-76). SARS is an infectious disease with a high potential for transmission to close contacts. Symptoms of SARS include fever (> 38° Celsius), dry cough, shortness of breath or breathing difficulties, and changes in chest X-rays indicative of pneumonia. Other symptoms include headache, muscular stiffness, loss of appetite, malaise, confusion, rash and diarrhea. At present, there is no specific therapy available for the prevention or treatment of a SARS-associated coronavirus infection. Given the potential for spread of SARS-associated coronavirus and the lethality of SARS, there is a need for prophylactic and therapeutic therapies for the prevention, treatment and/or amelioration of SARS-associated coronavirus infection.

### 2.2. GLYCYRRHIZIN

Glycyrrhizin, the most prominent compound found in licorice (*Glycyhrrhizza glabra*), is a triterpene glycoside. *Glycyhrrhizza glabra* is native to Turkey, Iraq, Spain, Greece, and northern China. *Glycyhrrhizza glabra* and Glycyrrhizin have been used for thousands of years as sweetening and flavoring agents in foods and for treatment of a variety of health problems.

The structure of Glycyrrhizin encompasses a triterpene portion (glycyrrhetinic acid) and two iduronic acid residues. There is a long history of usage to treat illnesses such as peptic ulcer (Glycyrrhizin inhibits the enzymes15-hydroxy-prostaglandin dehydrogenase and delta-13-prostaglandin reductase); colds and other viral infections (Glycyrrhizin may stimulate interferon production and has reported expectorant/cough suppressant properties); microbial and parasitic infections (Glycyrrhizin may stimulate immune system); cancers (again, possibly related to immune system function). For a review, see Wendell, 1998, U.S. Pharmacist 23(4), Herbal Pharmacy: Licorice. Glycyrrhizin inhibits the enzyme which breaks down cortisol; this prolongs the effects of naturally produced cortisol in the body, leading to anti-inflammatory effects as well as to sodium retention, water retention and potassium loss caused by glucocorticoids.

Other names for Glycyrrhizin include Glycyrrhizinic acid, Glycyrrhizic acid, Glycyrrhetinic acid glycoside, and (3-beta,20-beta)-20-Carboxy-11-oxo-30-norolean-12-en-3-yl 2-O-beta-D-glucopyranuronosyl-alpha-D-glucopyranosiduronic acid.

Citation of any reference in Section 2 or any other section of this application is not an admission that the reference is prior art to the application.

### 3. SUMMARY OF THE INVENTION

The invention provides Glycyrrhizin-based therapies for the prevention, treatment, management or amelioration of viral infections (e.g., coronavirus infections, Hepatitis C virus infections, influenza virus infections and West Nile virus infections). In particular, the invention provides Glycyrrhizin-based therapies for the prevention, treatment, management or amelioration of a SARS-associated coronavirus infection or one or more symptoms thereof. More specifically, the invention provides prophylactic and therapeutic protocols for the prevention, treatment, management or amelioration of a viral infections (*e.g*., coronavirus infections, Hepatitis C virus infections, influenza virus infections and West Nile virus infections, and preferably a SARS-associated coronavirus infection) or one or more symptoms thereof, comprising administering to a subject in need thereof a prophylactically or therapeutically effective amount of Glycyrrhizin or a derivative thereof, and optionally, a prophylactically or therapeutically effective amount of a prophylactic or therapeutic agent other than Glycyrrhizin or a derivative thereof. Examples of prophylactic or therapeutic agents other than Glycyrrhizin or a derivative thereof that can be used to prevent, treat, manage or ameliorate viral infections (*e.g*., coronavirus infections, Hepatitis C virus infections, influenza virus infections and West Nile virus infections, and preferably a SARS-associated coronavirus infection) or a symptom thereof include, but are not limited to, an antiviral agent, an antibiotic, an immunomodulatory agent, an anti-inflammatory agent, and an antibody that immunospecifically binds to a viral antigen (*e.g*., a SARS-associated coronavirus antigen).

In a specific embodiment, the invention provides a method for preventing, treating, managing or ameliorating a SARS-associated coronavirus infection, said method comprising administering to a subject (preferably a human subject) in need thereof a prophylactically or therapeutically effective amount of Glycyrrhizin or a derivative thereof. In another embodiment, the invention provides a method for preventing, treating, managing or ameliorating a SARS-associated coronavirus infection, said method comprising administering to a subject (preferably a human subject) in need thereof a prophylactically or therapeutically effective amount of Glycyrrhizin or a derivative thereof and a prophylactically or therapeutically effective amount of a prophylactic or therapeutic agent other than Glycyrrhizin or a derivative thereof. In accordance with these embodiments, Glycyrrhizin or a derivative thereof is preferably purified.

The invention encompasses compositions for use in the prevention, treatment, management and/or amelioration of viral infections (*e.g*., coronavirus infections, Hepatitis C virus infections, influenza virus infections and West Nile virus infections, and preferably a SARS-associated coronavirus infection) or one or more symptoms thereof. In a specific embodiment, a composition comprises Glycyrrhizin or a derivative thereof. In another embodiment, a composition comprises a compound of formula I, see *infra*. In another embodiment, a composition comprises Glycyrrhizin or a derivative thereof and one or more prophylactic or therapeutic agents other than Glycyrrhizin or a derivative thereof. In another embodiment, a composition comprises a compound of formula I, *infra*, and one or more prophylactic or therapeutic agents. In accordance with these embodiments, the compositions may further comprise a carrier. Non-limiting examples of prophylactic or therapeutic agents include immunomodulatory agents, anti-inflammatory agents, anti-viral agents, antibiotics, and antibodies, proteins, polypeptides or peptides that immunospecifically bind to a SARS-associated coronavirus antigen.

In a specific embodiment, a pharmaceutical composition comprises a pharmaceutically acceptable carrier, an effective amount of Glycyrrhizin or a derivative thereof, and optionally, an effective amount of one or more prophylactic or therapeutic agents other than Glycyrrhizin or a derivative thereof. In accordance with this embodiment, the pharmaceutical composition is preferably sterile and in suitable form for the intended method of administration.

The invention provides protocols for the administration of an effective amount of Glycyrrhizin or a derivative thereof alone or in combination with an effective amount of one or more therapies, other than Glycyrrhizin or a derivative thereof, for the prevention, treatment, management, or amelioration of viral infections (*e.g*., coronavirus infections, Hepatitis C virus infections, influenza virus infections and West Nile virus infections, and preferably a SARS-associated coronavirus infection) or one or more symptoms thereof to a subject in need thereof. The therapies (*e.g*., prophylactic or therapeutic agents) of the combination therapies of the present invention can be administered concomitantly or sequentially to a subject. The therapies (*e.g.*, prophylactic or therapeutic agents) of the combination therapies of the present invention can also be cyclically administered. Cycling therapy involves the administration of a first therapy (*e.g*., a first prophylactic or therapeutic agent) for a period of time, followed by the administration of a second therapy (*e.g*., a second prophylactic or therapeutic agent) for a period of time and repeating this sequential administration, *i.e*., the cycle, in order to reduce the development of resistance to one of the therapies (*e.g*., prophylactic or therapeutic agents), to avoid or reduce the side effects of one of the therapies (*e.g*., prophylactic or therapeutic agents), and/or to improve the efficacy of the therapies.

The therapies (*e.g*., prophylactic or therapeutic agents) of the combination therapies of the invention can be administered to a subject concurrently. The term "concurrently" is not limited to the administration of therapies (*e.g*., prophylactic or therapeutic agents) at exactly the same time, but rather it is meant that Glycyrrhizin or a derivative thereof and another therapy(ies) are administered to a subject in a sequence and within a time interval such that the Glycyrrhizin or a derivative thereof can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (*e.g*., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In preferred embodiments, two or more therapies (*e.g*., prophylactic or therapeutic agents) are administered to a patient within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

The prophylactic or therapeutic agents (*e.g*., Glycyrrhizin or a derivative thereof), compositions, or combination therapies of the invention may be administered by any method of administration well-known to one of skill in the art including, but not limited to, parenteral administration (*e.g*., intradermal, intramuscular, intraperitoneal, intravenous, and subcutaneous administration), epidural administration, topical administration, pulmonary administration, and mucosal administration (*e.g*., intranasal and oral routes). In a specific embodiment, a prophylactic or therapeutic agent, or a pharmaceutical composition is administered subcutaneously, intramuscularly, topically or intravenously to a subject. In a preferred embodiment, a prophylactic or therapeutic agent, or a pharmaceutical composition is administered orally, intranasally, or by pulmonary administration to a subject. The prophylactic or therapeutic agents or pharmaceutical compositions can be administered systematically or locally.

In one embodiment, a prophylactic or therapeutic agent (*e.g*., Glycyrrhizin or a derivative thereof), a composition, or combination therapy of the invention is administered locally to the area in need of treatment to a subject; this may be achieved by, for example, and not by way of limitation, local infusion, by injection, or by means of an implant, said implant being of a porous, non-porous, or gelatinous material, including membranes, such as sialastic membranes, or fibers. In another embodiment, a prophylactic or therapeutic agent (*e.g*., Glycyrrhizin or a derivative thereof), a composition, or a combination therapy of the invention is delivered to a subject in a vesicle. In another embodiment, a prophylactic or therapeutic agent (*e.g*., Glycyrrhizin or a derivative thereof), a composition, or a combination therapy of the invention is delivered to a subject in a controlled release or sustained release system.

The invention provides methods for inhibiting or reducing viral infections (*e.g*., coronavirus infections, Hepatitis C virus infections, influenza virus infections and West Nile virus infections, and preferably a SARS-associated coronavirus infection), said method comprising contacting a cell with Glycyrrhizin or a derivative thereof. The invention also provides methods for inhibiting or reducing one or more stages of viral infections (*e.g*., coronavirus infections, Hepatitis C virus infections, influenza virus infections and West Nile virus infections, and preferably a SARS-associated coronavirus infection). Such stages of a viral infection include, but are not limited to, entry into a cell, replication of the virus, expression of viral gene products, production of viral particles and release of viral particles. The invention also provides methods for inhibiting or reducing the replication of viral infections (*e.g*., coronavirus infections, Hepatitis C virus infections, influenza virus infections and West Nile virus infections, and preferably a SARS-associated coronavirus infection), said method comprising contacting a cell with Glycyrrhizin or a derivative thereof. The invention further provides methods for inhibiting or reducing the production and/or release of a viral particle (*e.g*., a coronavirus particle, a Hepatitis C virus particle, an influenza virus particle and a West Nile virus particle, and preferably a SARS-associated coronavirus particle), said method comprising contacting a cell with Glycyrrhizin or a derivative thereof.

In a specific embodiment, Glycyrrhizin or a derivative thereof inhibits or reduces a viral infection (*e.g*., coronavirus infection, a Hepatitis C virus infection, an influenza virus infection and a West Nile virus infection, and preferably a SARS-associated coronavirus infection) by at least 25%, preferably, at least 30%, at least 35%, at least 40%, at least 50%, at least 55%, at least 60%, at least 65%, at least 70%, at least 75%, at least 80%, at least 85%, at least 90%, at least 95%, or at least 98% relative to a control such as PBS in an *in vitro* and/or an *in vivo* assay described herein or well-known to one of skill in the art. In another embodiment, Glycyrrhizin or a derivative thereof inhibits or reduces the replication of a virus (*e.g*., a coronavirus, a Hepatitis C virus, an influenza virus and a West Nile virus, and preferably a SARS-associated coronavirus) by at least 25%, preferably, at least 30%, at least 35%, at least 40%, at least 50%, at least 55%, at least 60%, at least 65%, at least 70%, at least 75%, at least 80%, at least 85%, at least 90%, at least 95%, or at least 98% relative to a control such as PBS in an *in vitro* and/or an *in vivo* assay described herein or well-known to one of skill in the art. In yet another embodiment, Glycyrrhizin or a derivative thereof inhibits or reduces the production and/or release of a viral particle (*e.g*., coronavirus particle, a Hepatitis C virus particle, an influenza virus particle and a West Nile virus particle, and preferably a SARS-associated coronavirus particle) by at least 25%, preferably, at least 30%, at least 35%, at least 40%, at least 50%, at least 55%, at least 60%, at least 65%, at least 70%, at least 75%, at least 80%, at least 85%, at least 90%, at least 95%, or at least 98% relative to a control such as PBS in an *in vitro* and/or an *in vivo* assay described herein or well-known to one of skill in the art.

The present invention also provides articles of manufacture comprising in a container a prophylactic or therapeutic agent (*e.g*., Glycyrrhizin or a derivative thereof), and optionally in the same container or a different container a therapy, a prophylactic or therapeutic agent other than Glycyrrhizin or a derivative thereof, for the use in the prevention, treatment, management, or amelioration of a viral infection (*e.g*., coronavirus infection, a Hepatitis C virus infection, an influenza virus infection and a West Nile virus infection, and preferably a SARS-associated coronavirus infection) or a symptom thereof. The articles of manufacture may further comprise instructions.

In certain embodiments, the invention provides Glycyrrhizin derivatives of Formula I: 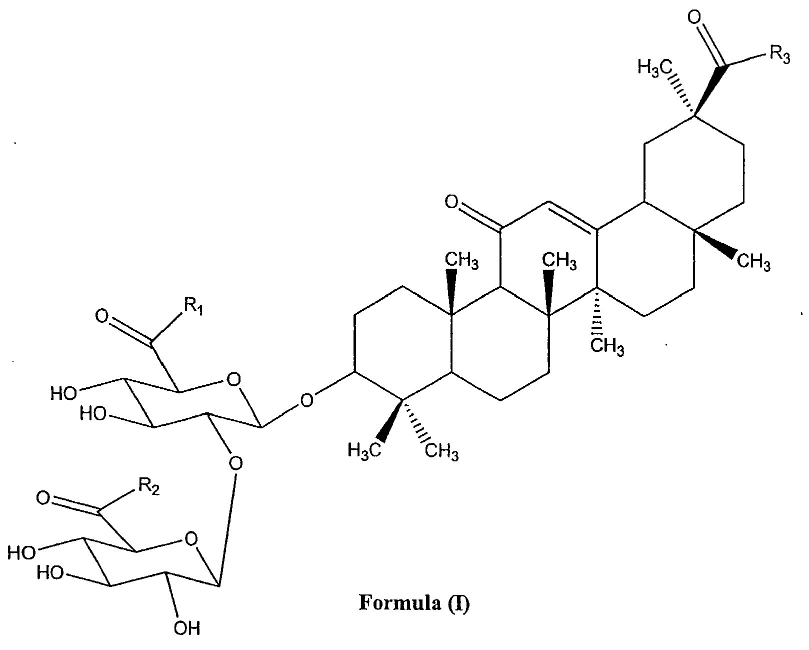 or a pharmaceutically acceptable salt thereof, wherein R₁, R₂, and R₃ are -N(H)R₄, wherein R₄ is -5-, 6-, or 7- membered heterocycle (substituted or unsubstituted), with the proviso that R₄ is not thiazole, uracil or

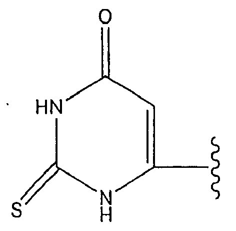

In certain embodiments, the invention provides compounds of Formula I: 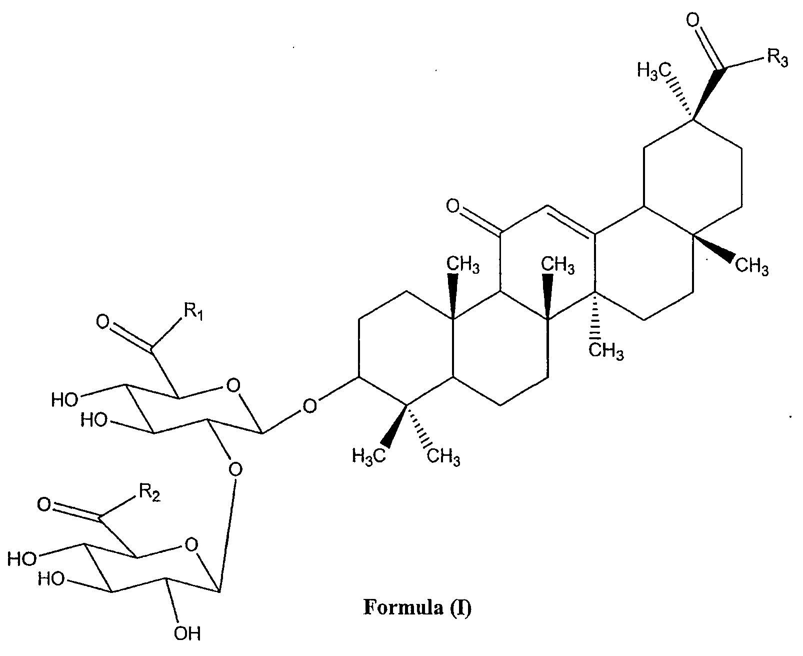 or a pharmaceutically acceptable salt thereof, wherein one of R₁ and R₂ is: 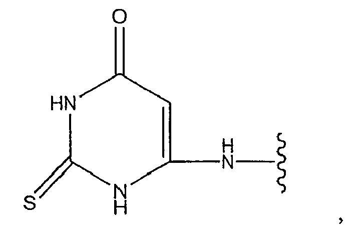 and R₃ and the other of R₁ and R₂ are independently -OH; -OCH₃; -NH-NH₂; -NHCH(COOH)CH₂SCH₂C₆H₅; 5-, 6-, or 7- membered heterocycle (substituted or unsubstituted); an amino acid; a peptide; -N(H)R₄, wherein R₄ is -5-, 6-, or 7- membered heterocycle (substituted or unsubstituted).

In certain embodiments, the invention provides Glycyrrhizin derivatives of Formula I: 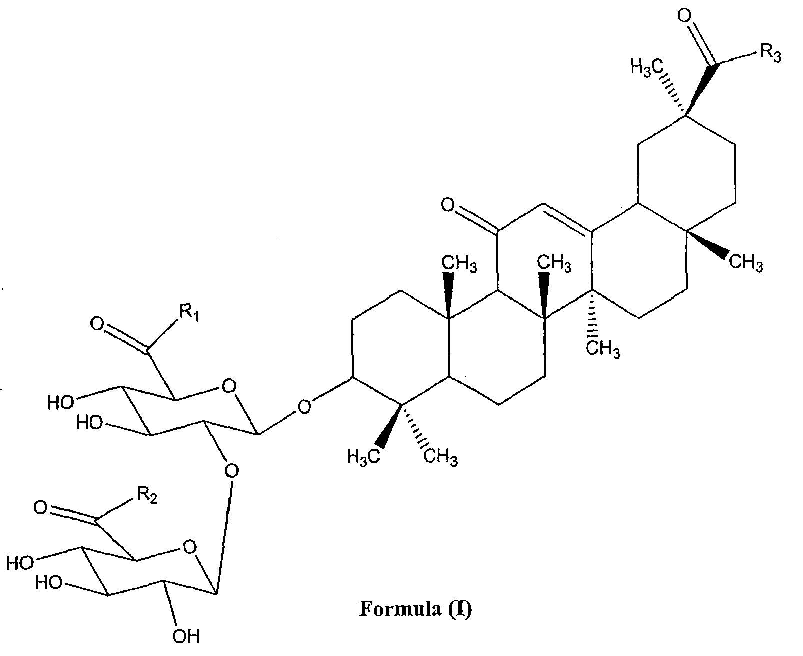 or a pharmaceutically acceptable salt thereof, wherein one of R₁, R₂, and R₃ is an amino acid or a peptide and the other two of R₁, R₂, and R₃ are independently -OH; -OCH₃; -NH-NH₂; -NHCH(COOH)CH₂SCH₂C₆H₅; 5-, 6-, or 7- membered heterocycle (substituted or unsubstituted); -N(H)R₄, wherein R₄ is -5-, 6-, or 7- membered heterocycle (substituted or unsubstituted).

In certain embodiments, the invention provides Glycyrrhizin derivatives of Formula I: 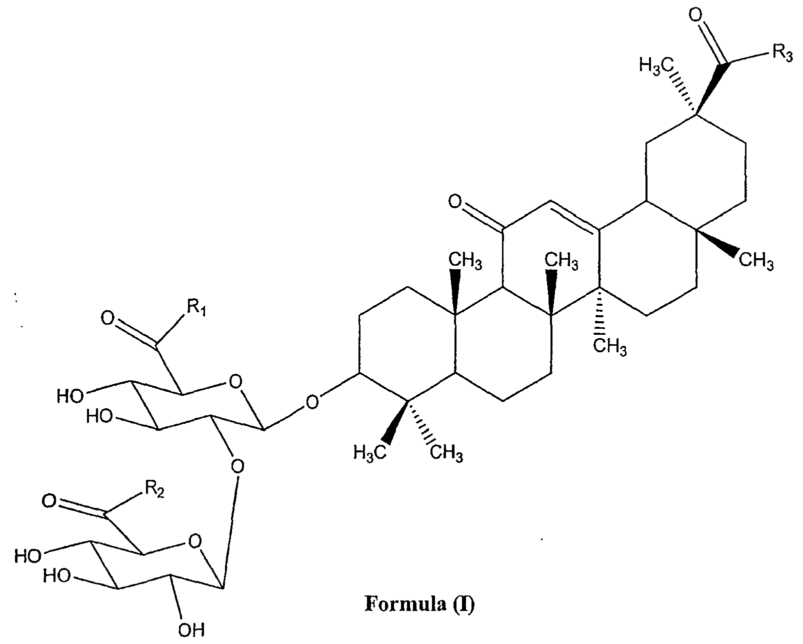 or a pharmaceutically acceptable salt thereof, wherein R₁, R₂, and R₃ are:

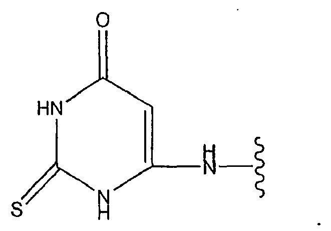

In certain embodiments, the invention provides Glycyrrhizin derivatives of Formula I: 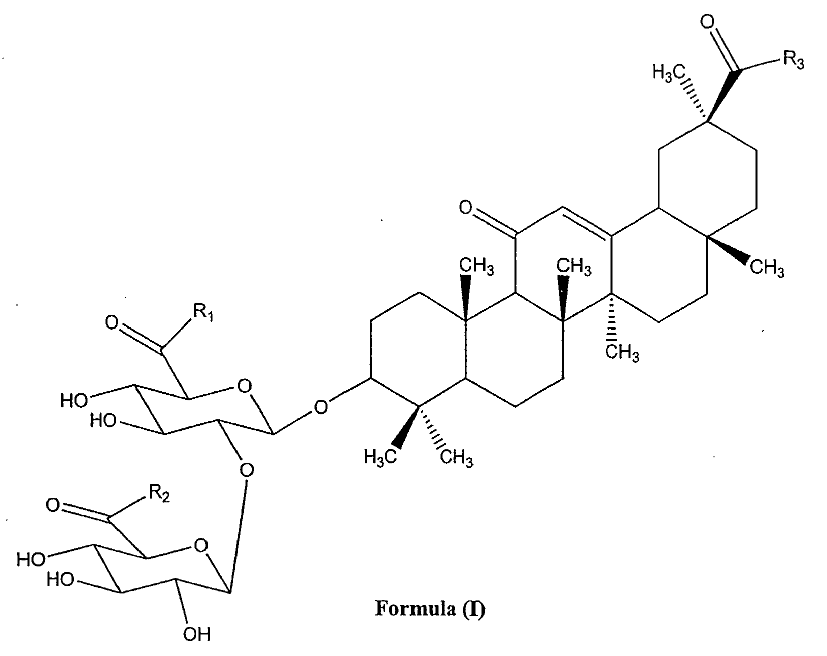 or a pharmaceutically acceptable salt thereof, wherein R₁, R₂, and R₃ are independently a 5-, 6-, or 7- membered heterocycle (substituted or unsubstituted), with the proviso that R₁, R₂, and R₃ are not all proline.

Glycyrrhizin derivatives of Formula (I) can be used to treat or prevent viral infections, e.g., infections with a coronavirus, such as SARS-associated coronavirus.

### 3.1. TERMINOLOGY & ABBREVIATIONS

Adjunctive: As used herein, the terms "adjunctive" and "conjunction" are used interchangeably with "in combination" or "combinatorial."

Antibody: As used herein, the terms "antibody" and "antibodies" refer to monoclonal antibodies, multispecific antibodies, human antibodies, humanized antibodies, camelised antibodies, chimeric antibodies, single-chain Fvs (scFv), single chain antibodies, single domain antibodies, Fab fragments, F(ab) fragments, disulfide-linked Fvs (sdFv), and anti-idiotypic (anti-Id) antibodies, and epitope-binding fragments of any of the above. In particular, antibodies include immunoglobulin molecules and immunologically active fragments of immunoglobulin molecules, *i.e.*, molecules that contain an antigen binding site. Immunoglobulin molecules can be of any type (*e.g.*, IgG, IgE, IgM, IgD, IgA and IgY), class (*e.g.*, IgG₁, IgG₂, IgG₃, IgG₄, IgA₁ and IgA₂) or subclass.

Antiviral Compound: As used herein, the terms "antiviral compound" and "antiviral agent" are used interchangeably.

Effective Amount: As used herein, the term "effective amount" refers to the amount of a therapy (*e.g*., a prophylactic or therapeutic agent) which is sufficient to reduce or ameliorate the severity and/or duration of a viral infection (*e.g*., coronavirus infection, a Hepatitis C virus infection, an influenza virus infection and a West Nile virus infection, and preferably a SARS-associated coronavirus infection) or one or more symptoms thereof, prevent the advancement of a viral infection (*e.g*., coronavirus infection, a Hepatitis C virus infection, an influenza virus infection and a West Nile virus infection, and preferably a SARS-associated coronavirus infection), prevent the recurrence, development, or onset of one or more symptoms associated with a viral infection (*e.g*., coronavirus infection, a Hepatitis C virus infection, an influenza virus infection and a West Nile virus infection, and preferably a SARS-associated coronavirus infection), prevent or reduce the replication or multiplication of a virus (*e.g*., coronavirus, a Hepatitis C virus, an influenza virus and a West Nile virus, and preferably a SARS-associated coronavirus), prevent or reduce the production and/or release of a viral particle (*e.g.*, coronavirus particle, a Hepatitis C virus particle, an influenza virus particle and a West Nile virus particle, and preferably a SARS-associated coronavirus particle), or enhance or improve the prophylactic or therapeutic effect(s) of another therapy (*e.g*., prophylactic or therapeutic agent). In a specific embodiment, an effective amount of a therapeutic or a prophylactic agent reduces one or more of the following steps of a the life cycle of a virus (*e.g*., coronavirus, a Hepatitis C virus, an influenza virus and a West Nile virus, and preferably a SARS-associated coronavirus): the docking of the virus particle to a cell, the introduction of viral genetic information into a cell, the expression of viral proteins, the production of new virus particles and the release of virus particles from a cell by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. In another specific embodiment, an effective amount of a therapeutic or a prophylactic agent reduces the replication, multiplication or spread of a virus by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. Non-limiting examples of effective amounts of Glycyrrhizin are described below.

Elderly Human: As used herein, the term "elderly human" refers to a human 65 years old or older, preferably 70 years old or older.

Human Child: As used herein, the term "human child" refers to a human between 24 months of age and 18 years of age.

Human Infant: As used herein, the term "human infant" refers to a human less than 24 months, preferably less than 16 months, less than 6 months, less than 3 months, less than 2 months, or less than 1 month of age.

Identity of Nucleic Acid Sequences: To determine the percent identity of two nucleic acid sequences, the sequences are aligned for optimal comparison purposes (*e.g*., gaps can be introduced in the sequence of a first amino acid or nucleic acid sequence for optimal alignment with a second amino acid or nucleic acid sequence). The nucleotides at corresponding amino acid positions or nucleotide positions are then compared. When a position in the first sequence is occupied by the same nucleotide as the corresponding position in the second sequence, then the molecules are identical at that position. The percent identity between the two sequences is a function of the number of identical positions shared by the sequences (*i.e*., % identity = number of identical overlapping positions/total number of positions x 100%). In one embodiment, the two sequences are the same length.

The determination of percent identity between two sequences can also be accomplished using a mathematical algorithm. A preferred, non-limiting example of a mathematical algorithm utilized for the comparison of two sequences is the algorithm of Karlin and Altschul, 1990, Proc. Natl. Acad. Sci. U.S.A. 87:2264-2268, modified as in Karlin and Altschul,1993, Proc. Natl. Acad. Sci. U.S.A. 90:5873-5877. Such an algorithm is incorporated into the NBLAST and XBLAST programs of Altschul et al., 1990, J. Mol. Biol. 215:403. BLAST nucleotide searches can be performed with the NBLAST nucleotide program parameters set, *e.g*., for score=1 00, wordlength=12 to obtain nucleotide sequences homologous to a nucleic acid molecules of the present invention. To obtain gapped alignments for comparison purposes, Gapped BLAST can be utilized as described in Altschul et al., 1997, Nucleic Acids Res. 25:3389-3402. Alternatively, PSI-BLAST can be used to perform an iterated search which detects distant relationships between molecules (Id.). When utilizing BLAST, Gapped BLAST, and PSI-Blast programs, the default parameters of the respective programs (*e.g.*, of XBLAST and NBLAST) can be used (see, *e.g.*, the NCBI website). Another preferred, non-limiting example of a mathematical algorithm utilized for the comparison of sequences is the algorithm of Myers and Miller, 1988, CABIOS 4:11-17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

Immunospecifically Binds to a viral antigen (*e.g*., coronavirus antigen, a Hepatitis C virus antigen, an influenza virus antigen and a West Nile virus antigen, and preferably a SARS-associated coronavirus antigen): As used herein, the term "immunospecifically binds to a a viral antigen (*e.g*., coronavirus antigen, a Hepatitis C virus antigen, an influenza virus antigen and a West Nile virus antigen, and preferably a SARS-associated coronavirus antigen)" and analogous terms refer to peptides, polypeptides, proteins, fusion proteins, and antibodies or fragments thereof that specifically bind to a virus (*e.g*., coronavirus, a Hepatitis C virus, an influenza virus and a West Nile virus, and preferably a SARS-associated coronavirus) and do not specifically bind to other polypeptides. A peptide, polypeptide, protein, or antibody that immunospecifically binds to a viral antigen (*e.g*., coronavirus antigen, a Hepatitis C virus antigen, an influenza virus antigen and a West Nile virus antigen, and preferably a SARS-associated coronavirus antigen) may bind to other peptides, polypeptides, or proteins with lower affinity as determined by, *e.g*., immunoassays, BIAcore, or other assays known in the art. Antibodies or fragments that immunospecifically bind to a viral antigen (*e.g*., coronavirus antigen, a Hepatitis C virus antigen, an influenza virus antigen and a West Nile virus antigen, and preferably a SARS-associated coronavirus antigen) may be cross-reactive with related antigens. Preferably, antibodies or fragments that immunospecifically bind to a a viral antigen (*e.g*., coronavirus antigen, a Hepatitis C virus antigen, an influenza virus antigen and a West Nile virus antigen, and preferably a SARS-associated coronavirus antigen) do not cross-react with other antigens. Antibodies or fragments that immunospecifically bind to a a viral antigen (*e.g*., coronavirus antigen, a Hepatitis C virus antigen, an influenza virus antigen and a West Nile virus antigen, and preferably a SARS-associated coronavirus antigen) can be identified, for example, by immunoassays, BIAcore, or other techniques known to those of skill in the art. An antibody or fragment thereof binds specifically to a viral antigen (*e.g*., coronavirus antigen, a Hepatitis C virus antigen, an influenza virus antigen and a West Nile virus antigen, and preferably a SARS-associated coronavirus antigen) when it binds to a viral antigen (*e.g*., coronavirus antigen, a Hepatitis C virus antigen, an influenza virus antigen and a West Nile virus antigen, and preferably a SARS-associated coronavirus antigen) with higher affinity than to any cross-reactive antigen as determined using experimental techniques, such as radioimmunoassays (RIA) and enzyme-linked immunosorbent assays (ELISAs). See, e.g., Paul, ed., 1989, Fundamental Immunology, 2nd ed., Raven Press, New York at pages 332-336 for a discussion regarding antibody specificity.

In Combination: As used herein, the term "in combination" refers to the use of more than one therapy (*e.g*., more than one prophylactic agent and/or therapeutic agent). The use of the term "in combination" does not restrict the order in which therapies (*e.g*., prophylactic and/or therapeutic agents) are administered to a subject with a virus (*e.g*., coronavirus, a Hepatitis C virus, an influenza virus and a West Nile virus, and preferably a SARS-associated coronavirus). A first therapy (*e.g*., a first prophylactic or therapeutic agent) can be administered prior to (*e.g.*, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g*., a second prophylactic or therapeutic agent) to a subject with a viral infection (*e.g*., coronavirus infection, a Hepatitis C virus infection, an influenza virus infection and a West Nile virus infection, and preferably a SARS-associated coronavirus infection).

Infection: As used herein, the phrase "infection" includes the invasion by and/or multiplication of a virus in a cell or body tissue, and the pathological state resulting from the invasion by and multiplication of a virus. The invasion by and multiplication steps of a virus' life cycle include, but are not limited to, the following steps: the docking of the virus particle to a cell, the introduction of viral genetic information into a cell, the expression of viral proteins, the production of new virus particles and the release of virus particles from a cell.

Manage: As used herein, the terms "manage," "managing," and "management" refer to the beneficial effects that a subject derives from a therapy (*e.g*., a prophylactic or therapeutic agent), which does not result in a cure of a viral infection (*e.g*., coronavirus infection, a Hepatitis C virus infection, an influenza virus infection and a West Nile virus infection, and preferably a SARS-associated coronavirus infection). In certain embodiments, a subject is administered one or more therapies (*e.g*., one or more prophylactic or therapeutic agents) to "manage" a disease so as to prevent the progression or worsening of a viral infection (*e.g*., coronavirus infection, a Hepatitis C virus infection, an influenza virus infection and a West Nile virus infection, and preferably a SARS-associated coronavirus infection).

Non-responsive: As used herein, the terms "non-responsive" and refractory" describe patients treated with a currently available therapy (*e.g*., prophylactic or therapeutic agent) for a viral infection (*e.g*., coronavirus infection, a Hepatitis C virus infection, an influenza virus infection and a West Nile virus infection, and preferably a SARS-associated coronavirus infection) which is not clinically adequate to relieve one or more symptoms associated with the infection. Typically, such patients suffer from severe, persistently active infection and require additional therapy to ameliorate the symptoms associated with the infection.

Prevent: As used herein, the terms "prevent", "preventing" and "prevention" refer to the prevention of the recurrence, onset or development of one or more symptoms of a viral infection (*e.g*., coronavirus infection, a Hepatitis C virus infection, an influenza virus infection and a West Nile virus infection, and preferably a SARS-associated coronavirus infection) in a subject resulting from the administration of a therapy (*e.g*., a prophylactic or therapeutic agent), or the administration of a combination of therapies (*e.g*., a combination of prophylactic or therapeutic agents).

Prophylactic Agent: As used herein, the terms "prophylactic agent" and "prophylactic agents" refer to any agent(s) which can be used in the prevention of a viral infection. In certain embodiments, the term "prophylactic agent" refers to a Glycyrrhizin or a derivative thereof. In certain other embodiments, the term "prophylactic agent" does not refer a Glycyrrhizin or a derivative thereof.

Prophylactically Effective Amount: As used herein, the term "prophylactically effective amount" refers to the amount of a therapy (*e.g*., prophylactic agent such as Glycyrrhizin or a derivative thereof) which is sufficient to result in the prevention of the development, recurrence, or onset of a viral infection (*e.g*., coronavirus infection, a Hepatitis C virus infection, an influenza virus infection and a West Nile virus infection, and preferably a SARS-associated coronavirus infection) or one or more symptoms thereof, or to enhance or improve the prophylactic effect(s) of another therapy (*e.g*., a prophylactic agent). In a specific embodiment, a prophylactically effective amount of a prophylactic agent reduces one or more of the following steps of the life cycle of a virus (*e.g*., coronavirus, a Hepatitis C virus, an influenza virus and a West Nile virus, and preferably a SARS-associated coronavirus): the docking of the virus particle to a cell, the introduction of viral genetic information into a cell, the expression of viral proteins, the, production of new virus particles and the release of virus particles from a cell by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. In another specific embodiment, a prophylactically effective amount of a prophylactic agent reduces the replication, multiplication or spread of a virus by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. Non-limiting examples of prophylactically effective amounts of Glycyrrhizin are described below.

Purified: As used herein, the term "purified" refers to a prophylactic or therapeutic agent (*e.g*., Glycyrrhizin or a derivative thereof) substantially free of a different prophylactic or therapeutic agent. Preferably, a prophylactic or therapeutic agent is at least 60%, 65%, 70%, 75%, 80%, 85%, 90%, 95%, or 99% free of a second, different prophylactic or therapeutic agent. In a preferred embodiment, Glycyrrhizin or a derivative thereof is purified.

Prophylactic Protocol: As used herein, a "prophylactic protocol" refers to a regimen for dosing and timing the administration of one or more prophylactic agents.

Protocol: A used herein, a "protocol" includes dosing schedules and dosing regimens. The protocols herein are methods of use and include prophylactic and therapeutic protocols.

Respiratory Tract: As used herein, the phrase "respiratory tract" refers to all organs and regions of the body's openings (*e.g*., nose, mouth, eyes (including the tear ducts) and ears) to the pulmonary alveoli.

Side Effects: As used herein, the phrase "side effects" encompasses unwanted and adverse effects of a prophylactic or therapeutic agent. Adverse effects are always unwanted, but unwanted effects are not necessarily adverse. An adverse effect from a prophylactic or therapeutic agent might be harmful or uncomfortable or risky.

Subject: As used herein, the terms "subject" and "patient" are used interchangeably. As used herein, the terms "subject" and "subjects" refer to an animal, preferably a mammal including a non-primate (*e.g*., a cow, pig, horse, cat, dog, rat, and mouse) and a primate (*e.g*., a monkey, such as a cynomolgous monkey, chimpanzee, and a human), and more preferably a human. In one embodiment, the subject is a mammal, preferably a human, with a SARS-associated coronavirus infection. In another embodiment, the subject is a farm animal (*e.g*., a horse, pig, or cow) or a pet (*e.g*., a dog or cat) with a SARS-associated coronavirus infection. In another embodiment, the subject is a mammal, preferably a human, at risk of developing a SARS-associated coronavirus infection. In another embodiment, the subject is a human infant. In another embodiment, the subject is a human child or a human adult. In another embodiment, the subject is a human in an institution or group home, such as, but not limited to, a nursing home. In yet another embodiment, the subject is refractory or non-responsive to current therapies for the prevention, treatment, management or amelioration of a viral infection (*e.g*., coronavirus infection, a Hepatitis C virus infection, an influenza virus infection and a West Nile virus infection, and preferably a SARS-associated coronavirus infection).

Synergistic: As used herein, the term "synergistic" refers to a combination of Glycyrrhizin or a derivative thereof and another therapy (*e.g*., a prophylactic or therapeutic agent), which is more effective than the additive effects of any two or more single therapies (*e.g*., one or more prophylactic or therapeutic agents). A synergistic effect of a combination of therapies (*e.g*., a combination of prophylactic or therapeutic agents) permits the use of lower dosages of one or more of therapies (*e.g*., one or more prophylactic or therapeutic agents) and/or less frequent administration of said therapies to a subject with a viral infection (*e.g*., coronavirus infection, a Hepatitis C virus infection, an influenza virus infection and a West Nile virus infection, and preferably a SARS-associated coronavirus infection). The ability to utilize lower dosages of therapies (*e.g*., prophylactic or therapeutic agents) and/or to administer said therapies less frequently reduces the toxicity associated with the administration of said therapies to a subject without reducing the efficacy of said therapies in the prevention or treatment of a viral infection (*e.g*., coronavirus infection, a Hepatitis C virus infection, an influenza virus infection and a West Nile virus infection, and preferably a SARS-associated coronavirus infection). In addition, a synergistic effect can result in improved efficacy of therapies (*e.g*., prophylactic or therapeutic agents) in the prevention or treatment of a viral infection (*e.g*., coronavirus infection, a Hepatitis C virus infection, an influenza virus infection and a West Nile virus infection, and preferably a SARS-associated coronavirus infection). Finally, synergistic effect of a combination of therapies (*e.g*., prophylactic or therapeutic agents) may avoid or reduce adverse or unwanted side effects associated with the use of any single therapy.

Therapeutic Agent: As used herein, the terms "therapeutic agent" and "therapeutic agents" refer to any agent(s) which can be used in the prevention, treatment, management or amelioration of one or more symptoms of a viral infection. In certain embodiments, the term "therapeutic agent" refers to Glycyrrhizin or a derivative thereof. In other embodiments, the term "therapeutic agent" does not refer to a Glycyrrhizin or a derivative thereof.

Therapeutically Effective Amount: As used herein, the term "therapeutically effective amount" refers to that amount of the therapeutic agent which is sufficient to reduce the severity of a viral infection (*e.g*., coronavirus infection, a Hepatitis C virus infection, an influenza virus infection and a West Nile virus infection, and preferably a SARS-associated coronavirus infection), reduce the duration of a viral infection (*e.g*., coronavirus infection, a Hepatitis C virus infection, an influenza virus infection and a West Nile virus infection, and preferably a SARS-associated coronavirus infection), ameliorate one or more symptoms of a SARS-associated coronavirus infection, prevent the advancement of a viral infection (*e.g*., coronavirus infection, a Hepatitis C virus infection, an influenza virus infection and a West Nile virus infection, and preferably a SARS-associated coronavirus infection), cause regression of a viral infection (*e.g*., coronavirus infection, a Hepatitis C virus infection, an influenza virus infection and a West Nile virus infection, and preferably a SARS-associated coronavirus infection), or to enhance or improve the therapeutic effect(s) of another therapeutic agent. With respect to the treatment of a viral infection (*e.g*., coronavirus infection, a Hepatitis C virus infection, an influenza virus infection and a West Nile virus infection, and preferably a SARS-associated coronavirus infection), a therapeutically effective amount refers to the amount of a therapeutic agent sufficient to reduce or inhibit the replication of a virus, inhibit or reduce the infection of a cell with the virus, inhibit or reduce the production of the viral particles, inhibit or reduce the release of viral particles, inhibit or reduce the spread of the virus to other tissues or subjects, or ameliorate one or more symptoms associated with the infection. In a specific embodiment, a therapeutically effective amount of a therapeutic agent reduces one or more of the following steps of a the life cycle of a virus (*e.g*., coronavirus, a Hepatitis C virus, an influenza virus and a West Nile virus, and preferably a SARS-associated coronavirus): the docking of the virus particle to a cell, the introduction of viral genetic information into a cell, the expression of viral proteins, the production of new virus particles and the release of virus particles from a cell by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. In another specific embodiment, a therapeutically effective amount of a therapeutic agent reduces the replication, multiplication or spread of a virus by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. Non-limiting examples of therapeutically effective amounts of Glycyrrhizin are described below.

Therapeutic Protocol: As used herein, the term "therapeutic protocol" refers to a regimen for dosing and timing the administration of one or more therapeutic agents.

Therapies: As used herein, the terms "therapies" and "therapy" refer to any protocol(s), method(s), and/or agent(s) that can be used in the prevention, treatment, management, or amelioration of a viral infection (*e.g.*, coronavirus infection, a Hepatitis C virus infection, an influenza virus infection and a West Nile virus infection, and preferably a SARS-associated coronavirus infection) or one or more symptoms thereof. In certain embodiments, the terms "therapy" and "therapy" refer to anti-viral therapy, antibiotic therapy, biological therapy, supportive therapy, and/or other therapies useful in treatment, management, prevention, or amelioration of a respiratory condition or one or more symptoms thereof known to skilled medical personnel.

Treat: As used herein, the terms "treat", "treatment" and "treating" to the reduction or amelioration of the progression, severity, and/or duration of a viral infection (*e.g*., coronavirus infection, a Hepatitis C virus infection, an influenza virus infection and a West Nile virus infection, and preferably a SARS-associated coronavirus infection) or the amelioration of one or more symptoms thereof resulting from the administration of one or more therapies (including, but not limited to, the administration of one or more prophylactic or therapeutic agents). In specific embodiments, such terms refer to the reduction or inhibition of the replication of a virus (*e.g*., coronavirus, a Hepatitis C virus, an influenza virus and a West Nile virus, and preferably a SARS-associated coronavirus), the inhibition or reduction in the spread of a virus (*e.g*., coronavirus, a Hepatitis C virus, an influenza virus and a West Nile virus, and preferably a SARS-associated coronavirus) to other tissues or subjects, the inhibition or reduction of infection of a cell with a virus (*e.g*., coronavirus, a Hepatitis C virus, an influenza virus and a West Nile virus, and preferably a SARS-associated coronavirus), or the amelioration of one or more symptoms associated with a viral infection (*e.g*., coronavirus infection, a Hepatitis C virus infection, an influenza virus infection and a West Nile virus infection, and preferably a SARS-associated coronavirus infection).

As used herein, a "5 to 7-membered heterocycle" is a 5- to 7-membered aromatic or nonaromatic ring of carbon atoms and from 1 to 3 heteroatoms selected from oxygen, nitrogen and sulfur. Examples of 5- to 7-membered heterocycles include, but are not limited to, tetrahydrofuranyl, dioxolanyl, pyrrolidinyl, morpholinyl, piperidyl, piperazinyl, tetrahydropyranyl, pyrimidine-2,4(1H,3H)-dione, and 2,3-dihydro-2-thioxopyrimidin-4(1H)-one.

As used herein, the term "amino acid" means any naturally occurring amino acid and non-naturally occurring amino acid such as a D-amino acid. An amino acid can be substituted with a protecting group. Suitable protecting groups for amino and amido groups include acetyl, tert-butoxy-C(O)-, benzyloxy-C(O)-, and the like. Suitable protecting groups for hydroxy include benzyl and the like. Suitable protecting groups for carboxy moieties include benzyl, tert-butyl, and the like. Other suitable protecting groups are well known to those of ordinary skill in the art and include those found in T. W. Greene, Protecting Groups in Organic Synthesis, John Wiley & Sons, Inc. 1981.

As used herein, the term "peptide" is a sequence of two to six amino acids. In certain embodiments, a peptide is two, three, four, five, or six amino acids long.

When the groups described herein are said to be "substituted or unsubstituted," when substituted, they may be substituted with any desired substituent or substituents that do not adversely affect the desired activity of the compound. Examples of preferred substituents are those found in the exemplary compounds and embodiments disclosed herein, as well as halogen (chloro, iodo, bromo, or fluoro); C₁₋₆ alkyl; C₂₋₆ alkenyl; C₂₋₆ alkynyl; hydroxyl; C₁₋₆ alkoxyl; amino; nitro; thiol; thioether; imine; cyano; amido; phosphonato; phosphine; carboxyl; thiocarbonyl; sulfonyl; sulfonamide; ketone; aldehyde; ester; oxygen (=0); haloalkyl (e.g., trifluoromethyl); carbocyclic cycloalkyl, which may be monocyclic or fused or non-fused polycyclic (e.g., cyclopropyl, cyclobutyl, cyclopentyl, or cyclohexyl), or a heterocycloalkyl, which maybe monocyclic or fused or non-fused polycyclic (e.g., pyrrolidinyl, piperidinyl, piperazinyl, morpholinyl, or thiazinyl); carbocyclic or heterocyclic, monocyclic or fused or non-fused polycyclic aryl (e.g., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothiophenyl, or benzofuranyl); benzyloxy; amino (primary, secondary, or tertiary); -N(CH₃)₂; O-lower alkyl; O-aryl, aryl; aryl-lower alkyl; CO₂CH₃; -OCH₂CH₃; methoxy; CONH₂; OCH₂CONH₂; NH₂; SO₂NH₂; OCHF₂; CF₃; OCF₃; and such moieties may also be optionally substituted by a fused-ring structure or bridge, for example -OCH₂O-.

These substituents may optionally be further substituted with a substituent selected from such groups.

It should be noted that if there is a discrepancy between a depicted structure and a name given that structure, the depicted structure controls. In addition, if the stereochemistry of a structure or a portion of a structure is not indicated with, for example, bold or dashed lines, the structure or portion of the structure is to be interpreted as encompassing all stereoisomers of it.

### 4. BRIEF DESCRIPTION OF THE FIGURES

Figure 1. Effect of Glycyrrhizin on replication of SARS-associated coronavirus in Vero cells. Cells were fixed with 60 parts methanol and 40 parts acetone 72 hours after infection. Virus was detected in serum from the patient with SARS by peroxidase staining. (A) mock infected cells; (B) infected cells without treatment; (C) infected cells treated with 4,000 mg/L Glycyrrhizin; (D) infected cells treated with 1,000 mg/L Glycyrrhizin.
Figure 2. Effect of Glycyrrhizin and Ribavirin on replication of SARS-associated coronavirus in Vero cells. (A) infected cells without treatment; (B) infected cells treated with 50mg/L Ribavirin; (C) infected cells treated with 100 mg/L Glycyrrhizin; and (D) infected cells treated with 50mg/L Ribavirin and 100 mg/L Glycyrrhizin.

### 5. DETAILED DESCRIPTION OF THE INVENTION

The invention provides Glycyrrhizin-based therapies for the prevention, treatment, management or amelioration of a viral infection (*e.g*., coronavirus infection, a Hepatitis C virus infection, an influenza virus infection and a West Nile virus infection, and preferably a SARS-associated coronavirus infection) or one or more symptoms thereof. In particular, the invention provides prophylactic and therapeutic protocols for the prevention, treatment, management or amelioration of a viral infection (*e.g*., coronavirus infection, a Hepatitis C virus infection, an influenza virus infection and a West Nile virus infection, and preferably a SARS-associated coronavirus infection) or one or more symptoms thereof, comprising administering to a subject in need thereof a prophylactically or therapeutically effective amount of Glycyrrhizin or a derivative thereof, and optionally, a prophylactically or therapeutically effective amount of a prophylactic or therapeutic agent other than Glycyrrhizin or a derivative thereof.

The invention provides methods for inhibiting or reducing a viral infection (*e.g*., coronavirus infection, a Hepatitis C virus infection, an influenza virus infection and a West Nile virus infection, and preferably a SARS-associated coronavirus infection), said method comprising contacting a cell with Glycyrrhizin or a derivative thereof. The invention also provides methods for inhibiting or reducing the replication of a virus (*e.g*., coronavirus, a Hepatitis C virus, an influenza virus and a West Nile virus, and preferably a SARS-associated coronavirus), said method comprising contacting a cell with Glycyrrhizin or a derivative thereof. The invention further provides methods for inhibiting or reducing the production and/or release of a viral particle (*e.g*., coronavirus particle, a Hepatitis C virus particle, an influenza virus particle and a West Nile virus particle, and preferably a SARS-associated coronavirus particle), said method comprising contacting a cell with Glycyrrhizin or a derivative thereof.

The present invention provides for pharmaceutical compositions and articles of manufacture comprising Glycyrrhizin or a derivative thereof for use in the prevention, treatment, management, or amelioration of a viral infection (*e.g*., coronavirus infection, a Hepatitis C virus infection, an influenza virus infection and a West Nile virus infection, and preferably a SARS-associated coronavirus infection) or one or more symptoms thereof. The present invention also provides for pharmaceutical compositions and articles of manufacture comprising Glycyrrhizin or a derivative thereof and one or more prophylactic or therapeutic agents other than Glycyrrhizin or a derivative thereof for use in prevention, treatment, management, or amelioration of a viral infection (*e.g*., coronavirus infection, a Hepatitis C virus infection, an influenza virus infection and a West Nile virus infection, and preferably a SARS-associated coronavirus infection) or one or more symptoms thereof.

### 5.1. GLYCYRRHIZIN AND DERIVATIVES

The present invention relates to using Glycyrrhizin ("Compound **1**") of the structure shown below.

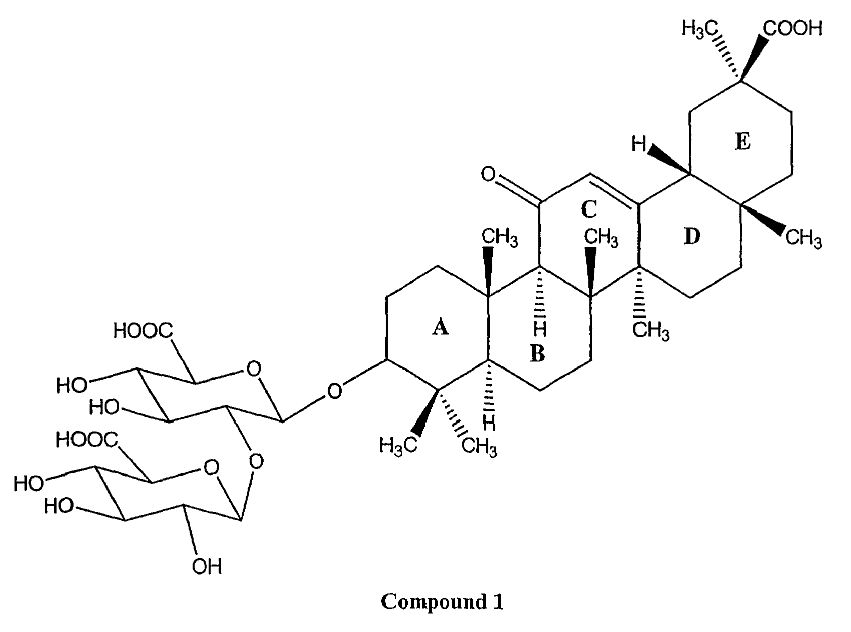

Derivatives of Glycyrrhizin include any compound wherein one or more of the carboxylic acid groups or hydroxyl groups of Glycyrrhizin have been modified (*i.e*., have undergone a synthetic transformation) or a pharmaceutically acceptable salt, free base, solvate, hydrate, stereoisomer, clathrate or prodrug thereof. In one embodiment, a Glycyrrhizin derivative is a compound wherein one, two or three carboxylic acid groups of Glycyrrhizin have been modified or a pharmaceutically acceptable salt, free base, solvate, hydrate, stereoisomer, clathrate or prodrug thereof. In another embodiment, a Glycyrrhizin derivative is a compound of formula I, including further embodiments thereof, or a pharmaceutically acceptable salt, free base, solvate, hydrate, stereoisomer, clathrate or prodrug thereof. In another embodiment, Glycyrrhizin derivatives include Compounds 2-8 or a pharmaceutically acceptable salt, free base, solvate, hydrate, stereoisomer, clathrate or prodrug thereof.

In certain embodiments, Glycyrrhizin or a derivative thereof is purified for use with the methods of the invention.

In a specific embodiment, a derivative of Glycyrrhizin is 18β-glycyrrhetinic acid. Without being bound by theory, 18β-glycyrrhetinic acid is the product of hydrolysis of Compound I catalyzed by the glucuronidase in the intestine. In another embodiment, a derivative of Glycyrrhizin is Glycyrrhizin sulfate.

Glycyrrhizin or a derivative thereof can be in the form of a or a pharmaceutically acceptable salt, free base, solvate, hydrate, stereoisomer, clathrate or prodrug thereof.

In certain embodiments, a derivative of Glycyrrhizin is a pharmaceutically acceptable salt of Glycyrrhizin. Pharmaceutically acceptable salts of Glycyrrhizin can be obtained by standard means. For example, an acid and Glycyrrhizin are dissolved in a solvent system in which both reactants (*i.e*., the free base of Glycyrrhizin and the respective acid) are sufficiently soluble. In one method, in order to achieve crystallization or precipitation, a solvent or solvent mixture in which the resulting salt is only slightly soluble or not soluble at all is used. Alternatively, a solvent in which the desired salt is very soluble can be used, and then an anti-solvent (or a solvent in which the resulting salt is poorly soluble) is added to the solution. Other variants for salt formation or crystallization includes concentrating the salt solution (e.g., by heating, under reduced pressure if necessary, or by slowly evaporating the solvent, for example, at room temperature), or seeding with the addition of seed crystals, or setting up water activity required for hydrate formation.

As used herein, the term "pharmaceutically acceptable salt(s)" refer to a salt prepared from a pharmaceutically acceptable non-toxic acid or base including an inorganic acid and base and an organic acid and base. Suitable pharmaceutically acceptable base addition salts for the compound of the present invention include, but are not limited to metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, methanesulfonic, and butyric acids. Other examples of specific salts include hydrochloride and mesylate salts. Others salts are well-known in the art, see for example, Remington's Pharmaceutical Sciences, 18th eds., Mack Publishing, Easton PA (1990) or Remington: The Science and Practice of Pharmacy, 19th eds., Mack Publishing, Easton PA (1995).

Pharmaceutically acceptable salts of Glycyrrhizin also include, but are not limited to, mono- or di-ammonium salts of Glycyrrhizin, mono- or di-sodium salts of Glycyrrhizin, mono- or di-potassium salts of Glycyrrhizin.

In certain embodiments, a derivative of Glycyrrhizin is a racemate and/or an enantiomer of Glycyrrhizin or a derivative of Glycyrrhizin.

In certain embodiments, Glycyrrhizin or a derivative thereof can be used in the form of a polymorph. As used herein and unless otherwise indicated, the term "polymorph" means a particular crystalline arrangement of the Glycyrrhizin or derivative thereof. Polymorphs can be obtained through the use of different work-up conditions and/or solvents. In particular, polymorphs can be prepared by recrystallization of Glycyrrhizin or derivative thereof in a particular solvent.

As used herein and unless otherwise indicated, the term "prodrug" means a Glycyrrhizin derivative that can hydrolyze, oxidize, or otherwise react under biological conditions (*in vitro* or *in vivo*) to provide an active compound, particularly a Glycyrrhizin or a derivative thereof. Examples of prodrugs include, but are not limited to, derivatives and metabolites of a Glycyrrhizin or a derivative thereof that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of Glycyrrhizin or a derivative thereof groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger's Medicinal Chemistry and Drug Discovery 6th ed. (Donald J. Abraham ed., 2001, Wiley) and Design and Application of Prodrugs (H. Bundgaard ed., 1985, Harwood Academic Publishers GmfH).

In certain embodiments, Glycyrrhizin or a derivative thereof is optically pure. As used herein and unless otherwise indicated, the term "optically pure" or "stereomerically pure" means one stereoisomer of a compound is substantially free of other stereoisomers of that compound. For example, a stereomerically pure compound having one chiral center will be substantially free of the opposite enantiomer of the compound. A stereomerically pure a compound having two chiral centers will be substantially free of other diastereomers of the compound. A typical stereomerically pure compound comprises greater than about 80% by weight of one stereoisomer of the compound and less than about 20% by weight of other stereoisomers of the compound, more preferably greater than about 90% by weight of one stereoisomer of the compound and less than about 10% by weight of the other stereoisomers of the compound, even more preferably greater than about 95% by weight of one stereoisomer of the compound and less than about 5% by weight of the other stereoisomers of the compound, and most preferably greater than about 97% by weight of one stereoisomer of the compound and less than about 3% by weight of the other stereoisomers of the compound.

In certain embodiments, to obtain a derivative of Glycyrrhizin, at least one of the -COOH groups of Compound I can be modified to be an ester, e.g., but not limited to, methylester, ethylester, n-propylester, iso-propylester.

In certain embodiments, to obtain a derivative of Glycyrrhizin, at least one of the six-membered rings in the triterpene portion of Compound I has one or more heteroatoms. Heteroatoms can be, but are not limited to, N, O, or P. In certain embodiments, the A ring, the B ring, the C ring, the D ring or the E ring is a heterocycle. In certain embodiments, at least two, at least three, at least four or all five of the six-membered rings in the triterpene portion are heterocycles.

In certain embodiments, to obtain a derivative of Glycyrrhizin, additional double bonds are introduced into the ring system of the triterpene portion. In certain embodiments, the ring system of the triterpene portion is aromatic.

In certain embodiments, to obtain a derivative of Glycyrrhizin, one or more of the -OH groups of Compound I can be modified to be acylated.

In certain embodiments, to obtain a derivative of Glycyrrhizin, at least one oxygen in Compound I is replaced by a sulfur.

In certain embodiments, a pharmaceutically acceptable salt of Glycyrrhizin is the monoammonium salt of Glycyrrhizin:

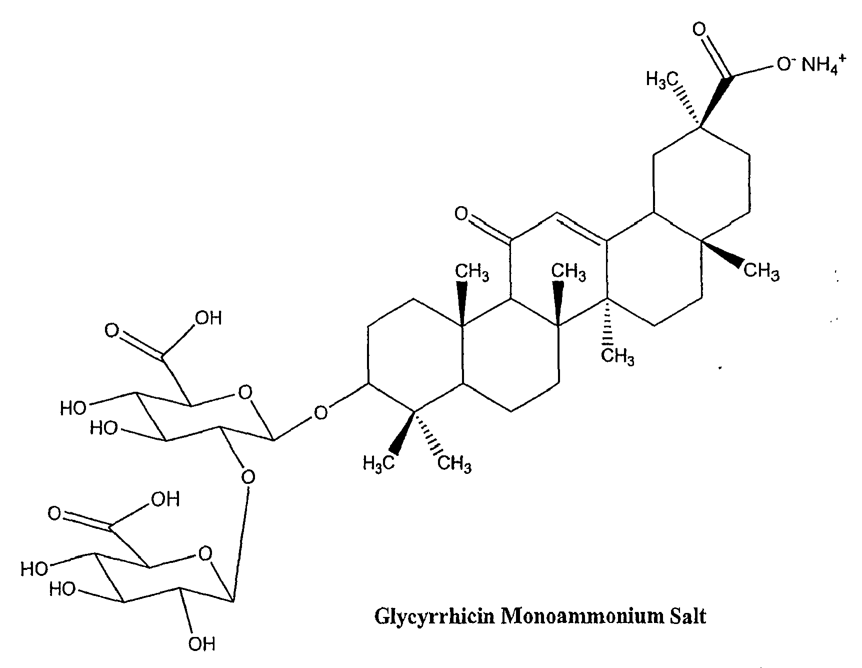

In certain embodiments, a derivative of Glycyrrhizin is a compound of Formula I 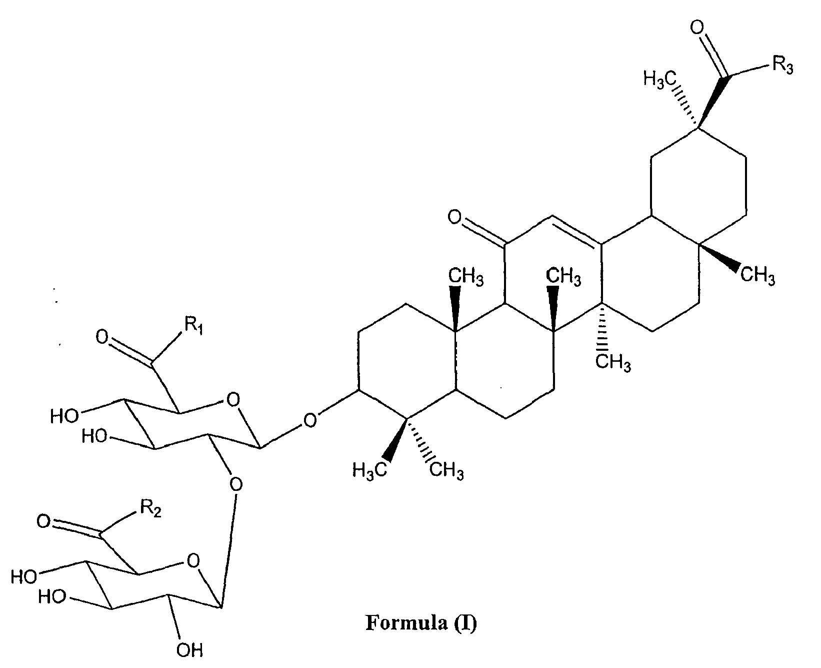 wherein R₁, R₂, and R₃ are independently: -OH; -OCH₃; NH-NH₂; -NHCH(COOH)CH₂SCH₂C₆H₅; 5-, 6-, or 7- membered heterocycle (substituted or unsubstituted); an amino acid; a peptide; or -N(H)R₄, wherein R₄ is -5-, 6-, or 7- membered heterocycle (substituted or unsubstituted). A further embodiment of Formula (I) is that wherein R₁, R₂, and R₃ are independently: -OH; 5-, 6-, or 7- membered heterocycle (substituted or unsubstituted); -Glycine-Leucine; or -N(H)R₄, wherein R₄ is -5-, 6-, or 7-membered heterocycle (substituted or unsubstituted)

In a further embodiment, the invention provides compounds of Formula (I) wherein R₁, R₂, and R₃ are -N(H)R₄, wherein each occurrence of R₄ is independently -5-, 6-, or 7-membered heterocycle (substituted or unsubstituted), with the proviso that R₄ is not thiazole, uracil or

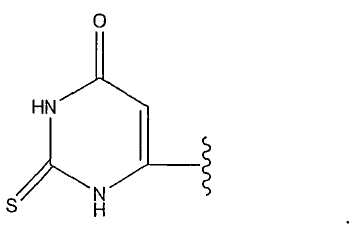

In a further embodiment, the invention provides compounds of Formula (I) wherein R₁, R₂, and R₃ are -N(H)R₄, wherein each occurrence of R₄ is independently a -5- membered heterocycle (substituted or unsubstituted), with the proviso that R₄ is not thiazole.

In a further embodiment, the invention provides compounds of Formula (I) wherein R₁, R₂, and R₃ are -N(H)R₄, wherein each occurrence of R₄ is independently a -6- membered heterocycle (substituted or unsubstituted), with the proviso that R₄ is not uracil or

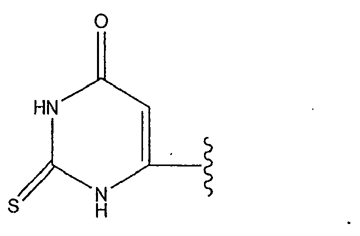

In a further embodiment, the invention provides compounds of Formula (I) wherein one of R₁ and R₂ is: 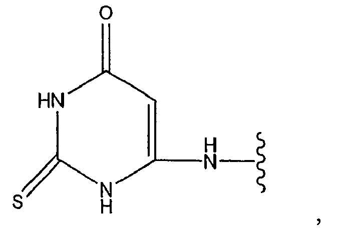 and R₃ and the other of R₁ and R₂ are independently -OH; -OCH₃; -NH-NH₂; -NHCH(COOH)CH₂SCH₂C₆H₅; 5-, 6-, or 7- membered heterocycle (substituted or unsubstituted); an amino acid; a peptide; -N(H)R₄, wherein R₄ is -5-, 6-, or 7- membered heterocycle (substituted or unsubstituted). In a still further embodiment, R₃ and the other of R₁ and R₂ are independently -OH; 5-, 6-, or 7- membered heterocycle (substituted or unsubstituted); -Glycine-Leucine; -N(H)R₄, wherein R₄ is -5-, 6-, or 7- membered heterocycle (substituted or unsubstituted).

In a further embodiment, the invention provides compounds of Formula (I) wherein one of R₁, R₂, and R₃ is an amino acid or a peptide and the other two of R₁, R₂, and R₃ are independently -OH; -OCH₃; -NH-NH₂; -NHCH(COOH)CH₂SCH₂C₆H₅; 5-, 6-, or 7-membered heterocycle (substituted or unsubstituted); -N(H)R₄, wherein R₄ is -5-, 6-, or 7-membered heterocycle (substituted or unsubstituted). In a still further embodiment, the other two of R₁, R₂, and R₃ are independently -OH; 5-, 6-, or 7- membered heterocycle (substituted or unsubstituted); -N(H)R₄, wherein R₄ is -5-, 6-, or 7- membered heterocycle (substituted or unsubstituted).

In a further embodiment, the invention provides compounds of Formula (I) wherein one of R₁, R₂, and R₃ is -Glycine-Leucine and the other two of R₁, R₂, and R₃ are independently -OH; -OCH₃; -NH-NH₂; -NHCH(COOH)CH₂SCH₂C₆H₅; 5-, 6-, or 7-membered heterocycle (substituted or unsubstituted); -N(H)R₄, wherein R₄ is -5-, 6-, or 7-membered heterocycle (substituted or unsubstituted). In a still further embodiment, the other two of R₁, R₂, and R₃ are independently -OH; 5-, 6-, or 7- membered heterocycle (substituted or unsubstituted); -N(H)R₄, wherein R₄ is -5-, 6-, or 7- membered heterocycle (substituted or unsubstituted).

In a further embodiment, the invention provides compounds of Formula (I) wherein two of R₁, R₂, and R₃ are -Glycine-Leucine and the other of R₁, R₂, and R₃ is -OH; -OCH₃; -NH-NH₂; -NHCH(COOH)CH₂SCH₂C₆H₅; 5-, 6-, or 7- membered heterocycle (substituted or unsubstituted); -N(H)R₄, wherein R₄ is -5-, 6-, or 7- membered heterocycle (substituted or unsubstituted). In a still further embodiment, the other of R₁, R₂, and R₃ is -OH; 5-, 6-, or 7-membered heterocycle (substituted or unsubstituted); -N(H)R₄, wherein R₄ is -5-, 6-, or 7-membered heterocycle (substituted or unsubstituted).

In a further embodiment, the invention provides compounds of Formula (I) wherein R₁, R₂, and R₃ are:

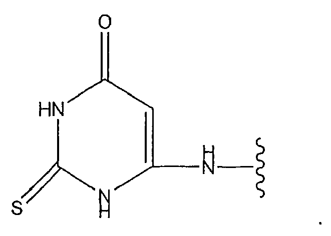

In a further embodiment, the invention provides compounds of Formula (I) wherein R₁, R₂, and R₃ are independently a 5-, 6-, or 7- membered heterocycle (substituted or unsubstituted), with the proviso that R₁, R₂, and R₃ are not all proline.

In certain, more specific embodiments, the invention provides derivatives of Glycyrrhizin of the following structures or pharmaceutically acceptable salts thereof:
Compound **2**: R₁, R₂, and R₃ each is 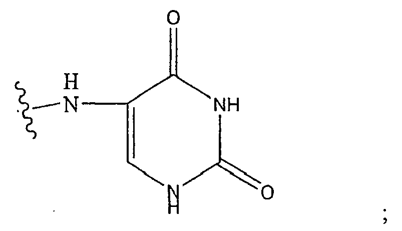
Compound **3**: R₃ is -OH; and R₁ and R₂ each is -Glycine-Leucine.
Compound **4**: R₃ is 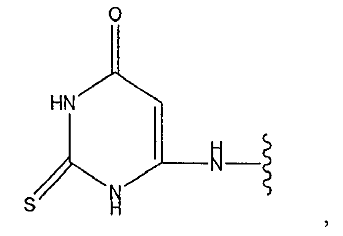 and R₁ and R₂ each is -OH;
Compound **5**: R₃ is -OH, and R₁ and R₂ each is 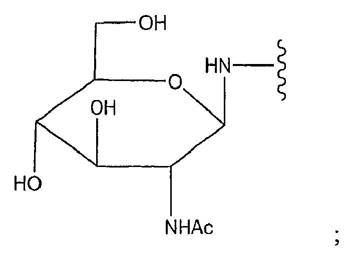
Compound **6**: R₁, R₂, and R₃ each is: 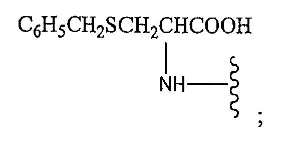
Compound **7**: 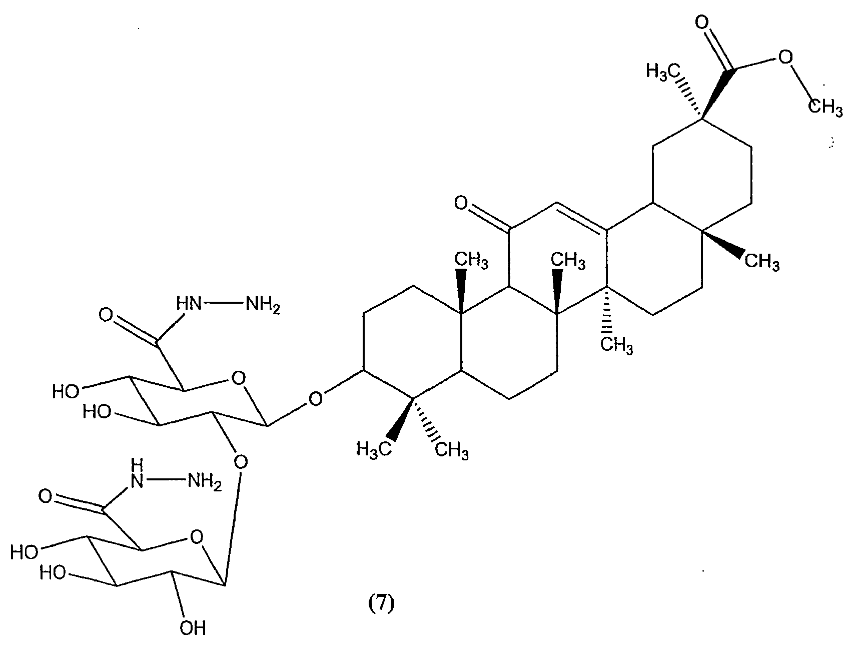
Compound **8**:

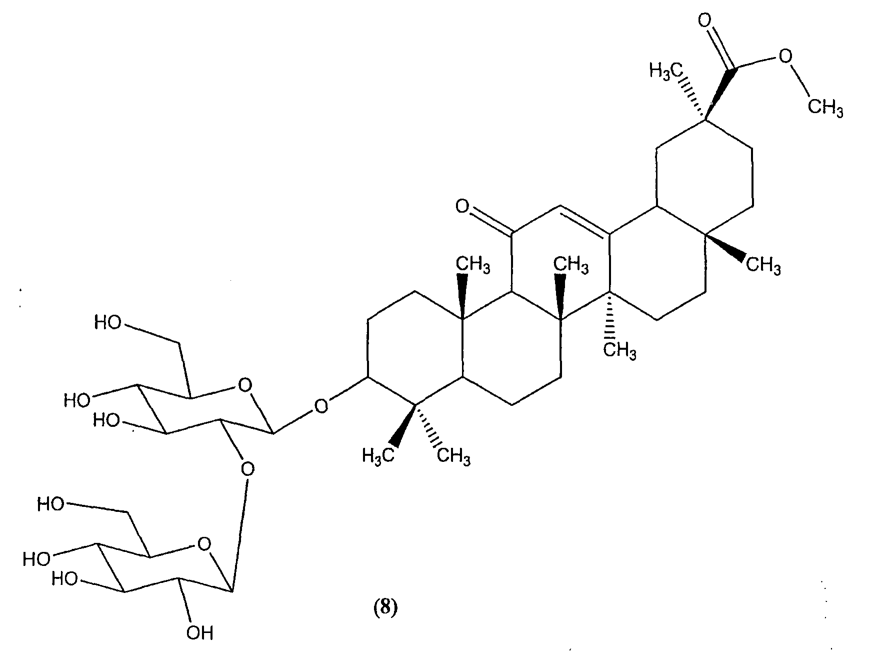

In certain embodiments, a Glycyrrhizin derivative of the invention has a an EC₅₀ of less than 3,000 mg/L, less than 1,500 mg/L, less than 500 mg/L, less than 250 mg/L, less than 100 mg/L, less than 50 mg/L, less than 10 mg/L or less than 1 mg/L. The EC₅₀ can be determined as described section 6.1 below.

In certain embodiments, a Glycyrrhizin derivative that can be used with the methods of the invention has a an EC₅₀ of less than 3,000 mg/L, less than 1,500 mg/L, less than 500 mg/L, less than 250 mg/L, less than 100 mg/L, less than 50 mg/L, less than 10 mg/L or less than 1 mg/L. The EC₅₀ can be determined as described section 6.1 below.

### 5.1.1 SYNTHESIS OF GLYCYRRHIZIN DERIVATIVES

The Glycyrrhizin derivatives can be obtained via standard, well-known synthetic methodology, *see e.g.* March, J. Advanced Organic Chemistry; Reactions Mechanisms, and Structure, 4th ed., 1992. Starting materials useful for preparing the compounds of the invention and intermediates therefore, are commercially available or can be prepared from commercially available materials using known synthetic methods and reagents.

Illustrative methods for the synthesis of Glycyrrhizin derivatives are described in Baltina, 2003, Current Medicinal Chemistry 10(2):155-171 which is incorporated herein by reference in its entirety.

### 5.2. AGENTS USEFUL IN COMBINATION WITH GLYCYRRHIZIN

Therapeutic or prophylactic agents that can be used in combination with Glycyrrhizin or a derivative thereof for the prevention, treatment, management or amelioration of a viral infection (*e.g*., coronavirus infection, a Hepatitis C virus infection, an influenza virus infection and a West Nile virus infection, and preferably a SARS-associated coronavirus infection) include, but are not limited to, small molecules, synthetic drugs, peptides (including cyclic peptides), polypeptides, proteins, nucleic acids (*e.g*., DNA and RNA nucleotides including, but not limited to, antisense nucleotide sequences, triple helices, RNAi, and nucleotide sequences encoding biologically active proteins, polypeptides or peptides), antibodies, synthetic or natural inorganic molecules, mimetic agents, and synthetic or natural organic molecules. Specific examples of such agents include, but are not limited to, immunomodulatory agents (*e.g*., interferon), anti-inflammatory agents (*e.g*., adrenocorticoids, corticosteroids (*e.g*., beclomethasone, budesonide, flunisolide, fluticasone, triamcinolone, methlyprednisolone, prednisolone, prednisone, hydrocortisone), glucocorticoids, steroids, and non-steriodal anti-inflammatory drugs (*e.g*., aspirin, ibuprofen, diclofenac, and COX-2 inhibitors)), pain relievers, leukotreine antagonists (*e.g.*, montelukast, methyl xanthines, zafirlukast, and zileuton), beta2-agonists (*e.g*., albuterol, biterol, fenoterol, isoetharie, metaproterenol, pirbuterol, salbutamol, terbutalin formoterol, salmeterol, and salbutamol terbutaline), anticholinergic agents (*e.g*., ipratropium bromide and oxitropium bromide), sulphasalazine, penicillamine, dapsone, antihistamines, anti-malarial agents (*e.g*., hydroxychloroquine), anti-viral agents (*e.g*., nucleoside analogs (*e.g*., zidovudine, acyclovir, gangcyclovir, vidarabine, idoxuridine, trifluridine, and ribavirin), foscarnet, amantadine, rimantadine, saquinavir, indinavir, ritonavir, and AZT) and antibiotics (*e.g*., dactinomycin (formerly actinomycin), bleomycin, erythomycin, penicillin, mithramycin, and anthramycin (AMC)).

In a specific embodiment, one or more of the following agents or a combination of the following agents are used in combination with Glycyrrhizin or a derivative thereof to prevent, treat, manage or ameliorate a viral infection (*e.g*., coronavirus infection, a Hepatitis C virus infection, an influenza virus infection and a West Nile virus infection, and preferably a SARS-associated coronavirus infection) or a symptom thereof: amantadine, ribavirin, rimantadine, acyclovir, famciclovir, foscamet, ganciclovir, trifluridine, vidarabine, didanosine, stavudine, zalciltabine, zidovudine, interferon (*e.g*., interferon-α, interferon-β, and/or interferon-γ), an antibiotic, an immunomodulatory agent, and an antibody or a fragment thereof (*e.g*., a human, a chimeric, a humanized, a camelized antibody, a monoclonal antibody, a polyclonal antibody, single chain antibody, SFv, a Fab fragment, and an F(ab') fragment) that immunospecifically binds to a viral antigen (*e.g*., coronavirus antigen, a Hepatitis C virus antigen, an influenza virus antigen and a West Nile virus antigen, and preferably a SARS-associated coronavirus antigen; such as, e.g., nonstructural protein, hemagglutinin-esterase glycoprotein, spike glycoprotein, small membrane gene, membrane glycoprotein, and nucleoprotein).

Any therapy which is known to be useful, or which has been used or is currently being used for the prevention, management, treatment, or amelioration of a viral infection or a respiratory condition, in particular a viral respiratory condition, or one or more symptoms thereof can be used in combination with Glycyrrhizin or a derivative thereof in accordance with the invention described herein. See, *e.g.*, Gilman et al., Goodman and Gilman's: The Pharmacological Basis of Therapeutics, 10th ed., McGraw-Hill, New York, 2001; The Merck Manual of Diagnosis and Therapy, Berkow, M.D. et al. (eds.), 17th Ed., Merck Sharp & Dohme Research Laboratories, Rahway, NJ, 1999; Cecil Textbook of Medicine, 20th Ed., Bennett and Plum (eds.), W.B. Saunders, Philadelphia, 1996 for information regarding therapies (*e.g*., prophylactic or therapeutic agents) which have been or are currently being used for preventing, treating, managing, or ameliorating a respiratory condition or one or more symptoms thereof.

To test for synergistic effects between Glycyrrhizin or a derivative thereof and a second compound against SARS-associated coronavirus, influenza virus, Hepatitis C virus, or West-Nile-Virus, any cell culture system and or animal model system for the respective viruses can be used (see sections 5.6 and 6.1). Controls include cells/animals without treatment, and cells/animals with treatment with the individual compounds can be included.

### 5.3. TARGET INFECTIONS

The invention provides for Glycyrrhizin-based therapies for the prevention, treatment, management or amelioration an infection with the virus that has been identified as the causative agent of Severe Acute Respiratory Syndrome. In particular, the invention also provides Glycyrrhizin-based therapies for the prevention, treatment, management or amelioration of a SARS-associated coronavirus infection. In a specific embodiment, the Glycyrrhizin-based therapies are used to prevent, treat, manage, or ameliorate a SARS-associated coronavirus having the nucleic acid sequence of a strain in Table 1. In a specific embodiment, the Glycyrrhizin-based therapies are used to prevent, treat, manage or ameliorate an infection with a SARS-associated coronavirus that is 50 to 65%, preferably 65 to 80%, more preferably 75 to 85%, and most preferably 85 to 99% identical on the nucleic acid level to the nucleic acid sequence of one of the isolates of SARS-associated coronavirus referenced in Table 1. In another embodiment, Glycyrrhizin-based therapies are used to prevent, treat, manage or ameliorate an infection with a SARS-associated coronavirus that is 50 to 65%, preferably 65 to 80%, identical on a nucleic acid level to the bovine coronavirus described in Drosten et al., 2003, N Engl J Med 348:1967-1976.

In certain embodiments, Glycyrrhizin-based therapies are used to prevent, treat, manage or ameliorate an infection with a SARS-associated coronavirus whose genome or fragments thereof hybridizes under conditions of high stringency to the genome or fragments thereof of bovine coronavirus described in Drosten et al., 2003, N Engl J Med 348:1967-1976. By way of example and not limitation, procedures using such conditions of high stringency are as follows: Prehybridization of filters containing DNA is carried out for 8 h to overnight at 65°C in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65°C in prehybridization mixture containing 100 µg/ml denatured salmon sperm DNA and 5-20 X 10⁶ cpm of ³²P-labeled probe. Washing of filters is done at 37°C for 1 h in a solution containing 2X SSC, 0.01% PVP, 0.01% Ficoll, and 0.01% BSA. This is followed by a wash in 0.1X SSC at 50°C for 45 min before autoradiography. Other conditions of high stringency which may be used are well known in the art.

In certain embodiments, Glycyrrhizin-based therapies are used to prevent, treat, manage or ameliorate an infection with a SARS-associated coronavirus whose genome or fragments thereof hybridizes under conditions of moderate stringency to the genome or fragments thereof of bovine coronavirus described in Drosten et al., 2003, N Engl J Med 348:1967-1976. For example, but not limited to, procedures using such conditions of moderate stringency are as follows: Filters containing DNA are pretreated for 6 h at 55°C in a solution containing 6X SSC, 5X Denhart's solution, 0.5% SDS and 100 µg/ml denatured salmon sperm DNA. Hybridizations are carried out in the same solution and 5-20 X 10⁶ cpm ³²P-labeled probe is used. Filters are incubated in hybridization mixture for 18-20 h at 55°C, and then washed twice for 30 minutes at 60°C in a solution containing 1X SSC and 0.1% SDS. Filters are blotted dry and exposed for autoradiography. Other conditions of moderate stringency which may be used are well-known in the art. Washing of filters is done at 37°C for 1 h in a solution containing 2X SSC, 0.1% SDS.

In certain embodiments, Glycyrrhizin-based therapies are used to prevent, treat, manage or ameliorate an infection with a SARS-associated coronavirus whose genome or fragments thereof hybridizes under conditions of low stringency to the genome or fragments thereof of bovine coronavirus described in Drosten et al., 2003, N Engl J Med 348:1967-1976. By way of example and not limitation, procedures using such conditions of low stringency are as follows (see also Shilo and Weinberg, 1981, Proc. Natl. Acad. Sci. USA 78:6789-6792): Filters containing DNA are pretreated for 6 h at 40°C in a solution containing 35% formamide, 5X SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20 X 10⁶ cpm ³²P-labeled probe is used. Filters are incubated in hybridization mixture for 18-20 h at 40°C, and then washed for 1.5 h at 55°C in a solution containing 2X SSC, 25 mM Tris-HCl (pH 7.4), 5 mM EDTA, and 0.1 % SDS. The wash solution is replaced with fresh solution and incubated an additional 1.5 h at 60°C. Filters are blotted dry and exposed for autoradiography. If necessary, filters are washed for a third time at 65-68°C and reexposed to film. Other conditions of low stringency which may be used are well known in the art.

In accordance with these embodiments, the SARS-associated coronavirus preferably causes lymphopenia and mildly elevated aminotransferase levels.

**TABLE 1. GenBank Accession Numbers of Different Strains of SARS-Associated Coronavirus**

| Isolate of SARS-Associated Coronavirus/ Type of Sequence | GenBank Accession Number |
|---|---|
| SARS coronavirus, complete genome | NC_004718 |
| SARS coronavirus CUHK-W1, complete genome | AY278554 |
| SARS coronavirus ZJ01, complete genome | AY297028 |
| SARS coronavirus Taiwan JC-2003, RNA directed RNA polymerase, partial coding sequence | AY286402 |
| SARS coronavirus TOR2, complete genome | AY274119 |
| SARS coronavirus TW1, complete genome | AY291451 |
| SARS coronavirus Tor2 RNA polymerase 1b mRNA | AY271716 |
| SARS coronavirus isolate SIN2774, complete genome | AY283798 |
| SARS coronavirus isolate SIN2748, complete genome | AY283797 |
| SARS coronavirus isolate SIN2679, complete genome | AY283796 |
| SARS coronavirus isolate SIN2677, complete genome | AY283795 |
| SARS coronavirus isolate SIN2500, complete genome | AY283794 |
| SARS coronavirus CUHK-Su10, complete genome | AY282752 |
| SARS coronavirus Hong Kong/03/2003 RNA-directed RNA polymerase gene | AY268070 |
| SARS coronavirus BJ01, complete genome | AY278488 |
| SARS coronavirus BJ04, partial genome | AY279354 |
| SARS coronavirus Urbani, complete genome | AY278741 |
| SARS coronavirus BJ03, partial genome | AY278490 |
| SARS coronavirus GZ01, partial genome | AY278489 |
| SARS coronavirus BJ02, partial genome | AY278487 |
| SARS coronavirus Taiwan RNA-directed RNA polymerase (pol) gene | AY268049 |
| SARS coronavirus HKU-39849, complete genome | AY278491 |
| SARS coronavirus Vietnam strain 200300592 polymerase gene, partial coding sequence | AY269391 |

The invention provides Glycyrrhizin-based therapies to protect a subject from infection with a SARS-associated coronavirus or to treat subject infected with a SARS-associated coronavirus before any symptoms of SARS manifest themselves. The invention also provides Glycyrrhizin-based therapies to prevent a subject that has been or is in contact with another subject with a SARS-associated coronavirus infection from developing a SARS-associated coronavirus infection. The invention further provides Glycyrrhizin-based therapies to prevent SARS in a subject exposed to a SARS-associated coronavirus. Any method known to the skilled artisan can be used to detect SARS-associated coronavirus (see Section 5.5, *infra*).

In certain embodiments, the SARS-associated coronavirus infection to be prevented, treated, managed or ameliorated in accordance with the invention causes or is associated with one or more of the following symptoms in a human subject: high fever (> 38° Celsius), dry cough, shortness of breath or breathing difficulties, and changes in chest X-rays indicative of pneumonia. In accordance with these embodiments, a SARS-associated coronavirus infection may cause or be associated with one or more of the following additional symptoms: a headache, muscular stiffness, loss of appetite, malaise, confusion, rash and diarrhea.

The invention also provides for Glycyrrhizin-based therapies for the prevention, treatment, management or amelioration of a viral infection other than, or in addition to, a SARS-associated coronavirus infection. In certain, more specific, embodiments, the invention provides for Glycyrrhizin-based therapies for the prevention, treatment, management or amelioration of an infection with a virus such as, but not limited to, DNA viruses such as hepatitis type B and hepatitis type C virus; parvoviruses, such as adeno-associated virus and cytomegalovirus; papovaviruses such as papilloma virus, polyoma viruses, and SV40; adenoviruses; herpes viruses such as herpes simplex type I (HSV-I), herpes simplex type II (HSV-II), and Epstein-Barr virus; poxviruses, such as variola (smallpox) and vaccinia virus; and RNA viruses, such as human immunodeficiency virus type I (HIV-I), human immunodeficiency virus type II (HIV-II), human T-cell lymphotropic virus type I (HTLV-I), human T-cell lymphotropic virus type II (HTLV-II), influenza virus, measles virus, rabies virus, Sendai virus, picomaviruses such as poliomyelitis virus, coxsackieviruses, rhinoviruses, reoviruses, togaviruses such as rubella virus (German measles) and Semliki forest virus, arboviruses, and hepatitis type A virus. In certain, even more specific, embodiments, the invention provides for Glycyrrhizin-based therapies for the prevention, treatment, management or amelioration of a coronavirus infection, a Hepatitis C virus infection, an influenza virus infection and a West Nile virus infection, and preferably a SARS-associated coronavirus infection.

### 5.4. THERAPEUTIC AND PROPHYLACTIC METHODS

### 5.4.1 SARS-ASSOCIATED CORONAVIRUS INFECTIONS

The present invention provides methods of preventing, treating, managing or ameliorating a SARS-associated coronavirus infection or one or more symptoms thereof, said method comprising administering to a subject in need thereof Glycyrrhizin or a derivative thereof. In a specific embodiment, the invention provides a method of preventing, treating, managing or ameliorating a SARS-associated coronavirus infection or one or more symptoms thereof, said method comprising administering to a subject in need thereof a dose of a prophylactically or therapeutically effective amount of Glycyrrhizin or a derivative thereof. An infection with SARS-associated coronavirus can be diagnosed by any method known to the skilled artisan. For exemplary methods see Section 5.5, *infra*.

The present invention provides methods of preventing, treating, managing or ameliorating a SARS-associated coronavirus or one or more symptoms thereof, said methods comprising administering to a subject in need thereof Glycyrrhizin or a derivative thereof and one or more prophylactic or therapeutic agents other than Glycyrrhizin or a derivative thereof. In a specific embodiment, the invention provides a method of preventing, treating, managing or ameliorating a SARS-associated coronavirus infection or one or more symptoms thereof, said method comprising administering to a subject in need thereof a dose of a prophylactically or therapeutically effective amount of Glycyrrhizin or a derivative thereof, and a dose of a prophylactically or therapeutically effective amount of one or more prophylactic or therapeutic agents other than Glycyrrhizin.

The present invention provides methods of preventing a SARS-associated coronavirus infection, said method comprising administering to a subject at risk of being infected with a SARS-associated coronavirus Glycyrrhizin or a derivative thereof. In a specific embodiment, the invention provides a method of preventing a SARS-associated coronavirus infection, said method comprising administering to a subject at risk of being infected with a SARS-associated coronavirus a dose of a prophylactically or therapeutically effective amount of Glycyrrhizin or a derivative thereof.

The present invention provides methods of preventing a SARS-associated coronavirus infection, said methods comprising administering to a subject at risk of being infected with a SARS-associated coronavirus Glycyrrhizin or a derivative thereof and one or more prophylactic or therapeutic agents other than Glycyrrhizin or a derivative thereof. In a specific embodiment, the invention provides a method of preventing a SARS-associated coronavirus infection, said method comprising administering to a subject at risk of being infected with a SARS-associated coronavirus a dose of a prophylactically or therapeutically effective amount of Glycyrrhizin or a derivative thereof, and a dose of a prophylactically or therapeutically effective amount of one or more prophylactic or therapeutic agents other than Glycyrrhizin or a derivative thereof.

The components (*e.g*., prophylactic or therapeutic agents) of the combination therapies of the invention can be administered sequentially or concurrently. In a specific embodiment, the combination therapies of the invention comprise Glycyrrhizin or a derivative thereof and at least one other therapy (*e.g*., at least one other prophylactic or therapeutic agent) which has a different mechanism of action than Glycyrrhizin or a derivative thereof. In another embodiment, the combination therapies of the invention comprise Glycyrrhizin or a derivative thereof and at least one other therapy (*e.g*., at least one other prophylactic agent) which has the same mechanism of action as Glycyrrhizin or a derivative thereof. In certain embodiments, the combination therapies of the present invention improve the prophylactic or therapeutic effect(s) of Glycyrrhizin or a derivative thereof by functioning together with Glycyrrhizin or a derivative thereof to have an additive or synergistic effect. In certain embodiments, the combination therapies of the present invention reduce the side effects associated with the prophylactic or therapeutic agents.

The prophylactic or therapeutic agents of the combination therapies can be administered to a subject, preferably a human subject, in the same pharmaceutical composition. In alternative embodiments, the prophylactic or therapeutic agents of the combination therapies can be administered sequentially or concurrently to a subject in separate pharmaceutical compositions. The prophylactic or therapeutic agents may be administered to a subject by the same or different routes of administration.

In one embodiment, a pharmaceutical composition comprising Glycyrrhizin or a derivative thereof is administered to a subject, preferably a human, to prevent, treat, manage or ameliorate a SARS-associated coronavirus or one or more symptoms thereof. In another embodiment, a pharmaceutical composition comprising Glycyrrhizin or a derivative thereof and one or more derivatives thereof and a prophylactic or therapeutic agent other than Glycyrrhizin or a derivative thereof, is administered to a subject, preferably a human, to prevent, treat, manage or ameliorate SARS-associated coronavirus or one or more symptoms thereof.

In certain embodiments, Glycyrrhizin or a derivative thereof is administered to a subject prior to or after symptoms of SARS or a SARS-associated coronavirus infection manifest, or prior to or after diagnosis of a SARS-associated coronavirus infection. In particular embodiments, Glycyrrhizin or a derivative thereof is administered to a subject prophylactically, when, for example, the subject is at increased risk of a SARS-associated coronavirus infection, such as when the subject is immunocompromised or immunosuppressed, or there is an epidemic of a SARS-associated coronavirus infection, or the subject is traveling or in a location that poses a greater risk of a SARS-associated coronavirus infection. In other embodiments, a pharmaceutical composition of the invention is administered to a subject at an early phase of a SARS-associated coronavirus infection. In yet other embodiments, a pharmaceutical composition of the invention is administered to a subject at later stages of a SARS-associated coronavirus infection to, e.g., prevent the consequences of a progressive SARS-associated coronavirus infection in the subject affected or prevent the spread of the virus to others.

### 5.4.2 OTHER VIRAL INFECTIONS

The invention also provides methods for the treatment, prevention, management or amelioration of infections with a virus comprising administering to a patient in need of treatment an effective amount of Glycyrrhizin or a derivative thereof. In other embodiments, a method of treating, managing, ameliorating or preventing an infection with a virus comprises administering to a patient in need of treatment an effective amount of Glycyrrhizin or a derivative thereof in combination with another therapy (*e.g*., an anti-viral agent; see section 5.2). In other embodiments, a method of treating, managing, ameliorating or preventing an infection with a virus comprises administering to a patient in need of treatment an effective amount of Glycyrrhizin or a derivative thereof in combination with Ribavirin.

The invention also provides methods for the treatment, prevention, management or amelioration of infections with viruses other than SARS-associated coronavirus, such as other coronaviruses, Hepatitis C virus, influenza virus, and West Nile Virus. In certain embodiments of the invention a method of treating, managing, ameliorating or preventing an infection with Hepatitis C virus, influenza virus, and West Nile Virus comprises administering to a patient in need of treatment an effective amount of Glycyrrhizin or a derivative thereof. In other embodiments, a method of treating, managing, ameliorating or preventing an infection with Hepatitis C, influenza virus, and West Nile Virus comprises administering to a patient in need of treatment an effective amount of Glycyrrhizin or a derivative thereof in combination with Ribavirin.

The effectiveness of the Glycyrrhizin or a derivative thereof or of Glycyrrhizin or a derivative in combination with another therapy (*e.g*., Ribavirin) against Hepatitis C virus, influenza virus, and West Nile Virus can be tested using any animal model system known to the skilled artisan. In certain, more specific embodiments, the effectiveness of Glycyrrhizin or a derivative thereof or of Glycyrrhizin or a derivative thereof in combination with another therapy (*e.g*., Ribavirin) against Hepatitis C virus, influenza virus, and West Nile Virus can be tested by treating infected cells with different concentrations of Glycyrrhizin or a derivative thereof or of Glycyrrhizin or a derivative thereof in combination with another therapy (*e.g*., Ribavirin). The cytopathic effect of the viral infection is measured in the presence and in the absence of Glycyrrhizin or a derivative thereof, or of Glycyrrhizin or a derivative thereof in combination with another therapy (*e.g*., Ribavirin; see, *e.g*., sections 5.6 and 6.1).

In certain embodiments, the invention provides methods for the treatment, prevention, management, or amelioration of a viral infection where the viral infection is non-responsive to other treatments.

The effectiveness of the Glycyrrhizin or a derivative thereof or of Glycyrrhizin or a derivative in combination with another therapy (*e.g*., Ribavirin) against Hepatitis C virus, influenza virus, and West Nile Virus can be tested using any animal model system known to the skilled artisan. In certain, more specific embodiments, the effectiveness of Glycyrrhizin or a derivative thereof or of Glycyrrhizin or a derivative thereof in combination with another treatment (*e.g*., Ribavirin) against Hepatitis C virus, influenza virus, and West Nile Virus can be tested by treating infected mice with different concentrations of Glycyrrhizin or a derivative thereof or of Glycyrrhizin or a derivative thereof in combination with another therapy (*e.g.*, Ribavirin). The propagation of virus in the animal is measured in the presence and in the absence of Glycyrrhizin or a derivative thereof or of Glycyrrhizin or a derivative thereof in combination with another therapy (*e.g*., Ribavirin; see, e.g., sections 5.6 and 6.1). In more specific embodiments, BALB/c mice are intranasally inoculated with 10(4) tissue culture 50% infective dose (TCID50). Two days later lungs and nasal turbinates are removed and stored at -70°C. The frozen tissues are homogenized in cell culture medium virus titers are determined using Vero cell monolayers.

In certain embodiments, the invention also provides methods for treating, preventing, ameliorating, or managing coronaviruses other than SARS-associated coronaviruses, wherein such methods comprise administering an effective amount of Glycyrrhizin or a derivative thereof to a patient infected with such a coronavirus.

In certain, more specific embodiments, the methods of the invention can be used to treat, prevent, ameliorate or manage infections with transmissible gastroenteritis virus (TGEV), porcine respiratory and reproductive virus infectious bronchitis virus, feline coronaviruses (FECV), and feline infectious peritonitis virus (FIPV).

### 5.4.3. TARGET PATIENTS

In certain embodiments, Glycyrrhizin or a derivative thereof or a combination therapy of the invention is administered to a subject who is or was in close contact with a subject who has been diagnosed with SARS. In certain embodiments, Glycyrrhizin or a derivative thereof or a combination therapy of the invention is administered to a subject who traveled within the last 10 days, within the last 15 days, within the last 30 days, within the last 50 days, within the last 75 days, or within the last 100 days to an area reporting cases of SARS. An updated list of such areas can be found on the website of the World Health Organization.

In certain embodiments, Glycyrrhizin or a derivative thereof or a combination therapy of the invention is administered to a subject who has one or more of the symptoms that are characteristic of SARS. Such symptoms include, but are not limited to, high fever (> 38° Celsius), dry cough, shortness of breath or breathing difficulties, and changes in chest X-rays indicative of pneumonia. Additional symptoms associated with or characteristic SARS include, but are not limited to, headache, muscular stiffness, loss of appetite, malaise, confusion, rash and diarrhea.

In certain embodiments, Glycyrrhizin or a derivative thereof or a combination therapy of the invention is administered to an elderly human subject or an immunocompromised or immunosuppressed subject. In other embodiments, Glycyrrhizin or a derivative thereof or a combination therapy of the invention is administered to an infant human subject or a subject in a group home or institution. In yet other embodiments, Glycyrrhizin or a derivative thereof or a combination therapy of the invention is administered to a subject who is between 0 and 10 years of age, between 10 and 20 years of age, between 30 and 40 years of age, between 40 and 50 years of age, between 50 and 60 years of age, between 60 and 70 years of age, between 70 and 80 years of age, between 80 and 90 years of age, between 90 and 100 years of age, between 100 and 120 years of age.

In certain embodiments, Glycyrrhizin or a derivative thereof or a combination therapy of the invention is administered to a subject who is infected with another virus, preferably a respiratory virus. In other embodiments, Glycyrrhizin or a derivative thereof or a combination therapy of the invention is administered to a subject who has previously been infected with another respiratory virus. In yet other embodiments, Glycyrrhizin or a derivative thereof or a combination therapy of the invention is administered to a subject who is or has previously been suffering from pneumonia.

### 5.5. DIAGNOSIS OF VIRUS INFECTION

Samples (*e.g*., sputum, mucus, sera, nasal aspirate, throat swab, broncho-alveolar lavage or other types of body fluids) from patients can be obtained and tested for the presence of a virus (*e.g*., SARS-associated coronavirus) to diagnose a viral infection (*e.g*., SARS-associated coronavirus infection). In certain embodiments, samples containing intact cells can be directly processed, whereas isolates without intact cells should first be cultured on a permissive cell line. In an illustrative embodiment, cultured cell suspensions should be cleared by centrifugation at, e.g., 300xg for 5 minutes at room temperature, followed by a PBS, pH 7.4 wash under the same conditions. Cell pellets are resuspended in a small volume of PBS for analysis. Primary clinical isolates containing intact cells are mixed with PBS and centrifuged at 300xg for 5 minutes at room temperature. Mucus is removed from the interface with a sterile pipette tip and cell pellets are washed once more with PBS under the same conditions. Pellets are then resuspended in a small volume of PBS for analysis.

A virus infection can be diagnosed by any method known to the skilled artisan. Exemplary methods for diagnosing a viral infection (*e.g*., a SARS-associated coronavirus infection), but are not limited to, detection of a nucleotide sequence of the virus (*e.g*., a SARS-associated coronavirus), detection of an antigen of the virus, and antibodies or fragments thereof that immunospecifically bind to the virus (*e.g*., a SARS-associated coronavirus). Examples of nucleotide sequences of a SARS-associated coronavirus are described in Drosten et al. (2003, N Eng1 J Med 348:1967-1976; see, *e.g*., Figure 1), which is incorporated by reference herein in its entirety. The genomic sequence information of SARS-associated coronavirus is publicly available; for GenBank accession numbers of different isolates of SARS-associated coronavirus see, e.g., Table 1. These sequences are available from the webpage of the National Center for Biotechnology Information (NCBI). Such nucleotide sequences can be detected by any method known to the skilled artisan, such as, but not limited to, PCR, RT-PCR or Northern blot analysis using probes specific to the nucleotide sequence of a SARS-associated coronavirus. Specific primers and protocols to detect SARS-associated coronavirus are described on the webpage of the World Health Organization and on the webpage of the Bernhard Nocht Institute for Tropical Medicine in Hamburg, Germany.

The coding sequences of a virus (*e.g*., SARS-associated coronavirus) can be expressed to produce proteins, polypeptides or peptides of the virus (*e.g*., SARS-associated coronavirus proteins, polypeptides or peptides), which can be used to generate antibodies that detect the virus (*e.g*., SARS-associated coronavirus). Antibodies that immunospecifically bind to a virus (*e.g*., SARS-associated coronavirus antigen) can be used to detect a virus (*e.g*., SARS-associated coronavirus) using techniques well-known to those of skill in the art, such as immunoassays (*e.g*., immunoprecipitation, Western blots, ELISA and flow cytometry).

Immunoprecipitation protocols generally comprise lysing a population of cells in a lysis buffer such as RIPA buffer (1% NP-40 or Triton X-100, 1% sodium deoxycholate, 0.1 % SDS, 0.15 M NaCl, 0.01 M sodium phosphate at pH 7. 2, 1 % Trasylol) supplemented with protein phosphatase and/or protease inhibitors (*e.g*., EDTA, PMSF, 159 aprotinin, sodium vanadate), adding the antibody of interest to the cell lysate, incubating for a period of time (*e.g.*, to 4 hours) at 4 degrees C, adding protein A and/or protein G sepharose beads to the cell lysate, incubating for about an hour or more at 4 degrees C, washing the beads in lysis buffer and re-suspending the beads in SDS/sample buffer. The ability of the antibody of interest to immunoprecipitate a particular antigen can be assessed by, *e.g*., Western blot analysis. One of skill in the art would be knowledgeable as to the parameters that can be modified to increase the binding of the antibody to an antigen and decrease the background (*e.g*., pre-clearing the cell lysate with sepharose beads). For further discussion regarding immunoprecipitation protocols see, e.g., Ausubel et al., eds., 1994, Current Protocols in Molecular Biology, Vol. 1, John Wiley & Sons, Inc., New York at pages 10, 16, 1.

Western blot analysis generally comprises preparing protein samples, electrophoresis of the protein samples in a polyacrylamide gel (*e.g*., 8%- 20% SDS-PAGE depending on the molecular weight of the antigen), transferring the protein sample from the polyacrylamide get to a membrane such as nitrocellulose, PVDF or nylon, blocking the membrane, in blocking solution (*e.g*., PBS with 3% BSA or non-fat milk), washing the membrane in washing buffer (*e.g*., PBSTween20), incubating the membrane with primary antibody (the antibody of interest) diluted in blocking buffer, washing the membrane in washing buffer, incubating the membrane with a secondary antibody (which recognizes the primary antibody, e.g., an antihuman antibody) conjugated to an enzymatic substrate (e.g., horseradish peroxidase or alkaline phosphatase) or radioactive molecule (e.g., ¹²P or ¹²¹I) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding Western blot protocols see, e.g., Ausubel et al., eds., 1994, GinTent Protocols in Molecular Biology, Vol. 1, John Wiley & Sons, Inc., New York at 10.8.1.

ELISAs comprise preparing antigen, coating the well of a 96-well microtiter plate with the antigen, washing away antigen that did not bind the wells, adding the antibody of interest conjugated to a detectable compound such as an enzymatic substrate (*e.g*., horseradish peroxidase or alkaline phosphatase) to the wells and incubating for a period of time, washing away unbound antibodies or non-specifically bound antibodies, and detecting the presence of the antibodies specifically bound to the antigen coating the well. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, the detectable molecule could be the antigen conjugated to a detectable compound such as an enzymatic substrate (*e.g*., horseradish peroxidase or alkaline phosphatase). The parameters that can be modified to increase signal detection and other variations of ELISAs are well known to one of skill in the art. For further discussion regarding ELISAs *see*, *e.g.*, Ausubel et al., eds, 1994, Current Protocols in Molecular Biology, Vol. I, John Wiley & Sons, Inc., New York at 11.2.1.

The binding affinity of an antibody (including a scFv or other molecule comprising, or alternatively consisting of, antibody fragments or variants thereof) to an antigen and the off-rate of an antibody-antigen interaction can be determined by competitive binding assays. One example of a competitive binding assay is a radioimmunoassay comprising the incubation of labeled antigen (*e.g*., ³H or ¹²¹I) with the antibody of interest in the presence of increasing amounts of unlabeled antigen, and the detection of the antibody bound to the labeled antigen.

The presence of antibodies that immunospecifically bind to a virus (*e.g*., a SARS-associated coronavirus) can be detected in a subject to diagnose the presence of a virus (*e.g*., SARS-associated coronavirus) in the subject. Any method known to the skilled artisan can be used to detect the presence of antibodies that immunospecifically bind to a viral antigen (*e.g*., a SARS-associated coronavirus antigen; including immunoassays such as ELISAs).

In an illustrative embodiment, a SARS-associated coronavirus antigen are linked to a solid support. Subsequently, the material that is to be tested for the presence of antibodies that immunospecifically bind to a SARS-associated coronavirus antigen is incubated with the solid support under conditions conducive to the binding of the antibodies to a SARS-associated coronavirus antigen. Subsequently, the solid support is washed under conditions that remove any unspecifically bound antibodies. Following the washing step, the presence of bound antibodies can be detected using any technique known to the skilled artisan. In a specific embodiment, the SARS-associated coronavirus antigen-antibody complex is incubated with a detectably labeled antibody that recognizes antibodies that were generated by the species of the subject, *e.g*., if the subject is a human, the detectably labeled antibody is directed to human antibodies, under conditions conducive to the binding of the detectably labeled antibody to the antibody that is bound to the SARS-associated coronavirus antigen. In a specific embodiment, the detectably labeled antibody is conjugated to an enzymatic activity. In another embodiment, the detectably labeled antibody is radioactively labeled. The complex of SARS-associated coronavirus antigen-antibody-detectably labeled antibody is then washed, and subsequently the presence of the detectably labeled antibody is quantified by any technique known to the skilled artisan, wherein the technique used is dependent on the type of label of the detectably labeled antibody.

The incidence of infection can be determined by any method well-known in the art, for example, but not limited to, clinical samples (*e.g*., nasal swabs) can be tested for the presence of a virus (*e.g*., SARS-associated coronavirus) by immunofluorescence assay (IFA) using an anti-virus antigen antibody (*e.g*., anti- SARS-associated coronavirus -antigen antibody). In other embodiments, virus-specific (*e.g*., SARS-associated coronavirus specific) nucleotide sequences are detected by any method known in the art. Such methods include, but are not limited to, PCR, RT-PCR, and Northern blot hybridization.

### 5.6. BIOLOGICAL ASSAYS

Several aspects of the therapies (*e.g*., prophylactic or therapeutic agents) of the invention are preferably tested *in vitro*, in a cell culture system, and in an animal model organism, such as a rodent animal model system, for the desired therapeutic activity prior to use in humans. For example, assays which can be used to determine whether administration of Glycyrrhizin or a derivative thereof or a specific combination of Glycyrrhizin or a derivative thereof and another anti-viral compound is indicated, include cell culture assays in which a patient tissue sample is grown in culture, and exposed to or otherwise contacted with Glycyrrhizin or a derivative thereof, or Glycyrrhizin or a derivative thereof and another anti-viral compound and the effect of such agent(s) upon the tissue sample is observed. The tissue sample can be obtained by biopsy from the patient. This test allows the identification of the therapeutically most effective Glycyrrhizin or a derivatives thereof or combinations of Glycyrrhizin or a derivative thereof and other anti-viral compounds. In various specific embodiments, *in vitro* assays can be carried out with representative cells of cell types involved in a particular viral infection, such as, e.g., cells obtained from lung tissue.

The prophylactic or therapeutic agents can be assessed for their ability to alter viral replication (as determined, *e.g*., by plaque formation) or the production of viral proteins (as determined, *e.g*., by Western blot analysis, RT-PCR or Northern blot analysis) in cultured cells *in vitro* using methods which are well known in the art. Any method known to the skilled artisan can be used to test Glycyrrhizin and derivatives thereof for their effect on the ability of a virus (*e.g*., SARS-associated coronavirus) to infect a cell, to replicate in a cell, or to propagate in an host.

The viability of a virus (*e.g*., SARS-associated coronavirus) infected cell can be determined by any technique known to the skilled artisan. In certain embodiments, the proliferation of the virus-infected cell is measured to determine viability of the virus infected cell. Different cell types (including patient cells and cell lines) can be used for this assay, such as, but not limited to, Vero cells, HeLa cells, HeLa, 16HBE14o, HMEC-1,1301 and MOLT-4. The cell can be infected with a SARS-associated coronavirus.

In certain embodiments, the effect of Glycyrrhizin and derivatives thereof on the virus infected cell is determined by measuring the production of infectious virus particles by the virus-infected cell. In certain embodiments, samples of the medium in which the infected cells are grown are taken at different time points after infection, such as 4 hours, 12 hours, 24 hours, 48 hours and 72 hours after infection. The titer of the virus in the supernatant can be determined using a TCID₅₀ test.

In certain other embodiments, the production of viral proteins by the infected cell is determined. The level of viral proteins can be determined by SDS-PAGE and subsequent Western blot analysis using antibodies specific to the viral protein. Viral proteins include for example nonstructural proteins, hemagglutinin-esterase glycoprotein, spike glycoprotein, small membrane gene, membrane glycoprotein, and nucleoprotein.

The assays described herein may be used to assay viral titre over time to determine the growth characteristics of the virus in the presence and the absence of Glycyrrhizin or a derivative thereof or a combination of Glycyrrhizin or a derivative thereof and another anti-viral compound. In a specific embodiment, the viral titre is determined by obtaining a sample from the infected cells or the infected subject, preparing a serial dilution of the sample and infecting a monolayer of cells that are susceptible to infection with the virus at a dilution of the virus that allows for the emergence of single plaques. The plaques can then be counted and the viral titre express as plaque forming units per milliliter of sample. In a specific embodiment of the invention, the growth rate of a virus of the invention in a subject is estimated by the titer of antibodies against the virus in the subject. Samples from a subject can be obtained by any method known to the skilled artisan. In certain embodiments, the sample consists of nasal aspirate, throat swab, sputum or broncho-alveolar lavage.

In certain embodiments, survival of cells infected with a virus (e.g., SARS-associated coronavirus) is an indicator for the effectiveness of Glycyrrhizin or a derivative thereof or a combination of Glycyrrhizin or a derivative thereof and another anti-viral compound. Many assays well-known in the art can be used to assess cell survival and/or growth; for example, cell proliferation can, be assayed by measuring Bromodeoxyuridine (BRDU) incorporation (see, *e.g*., Hoshino et al., 1986, Int. J. Cancer 38, 369; Campana et al., 1988, J. Immunol. Meth, 107:79) or (³H)-thymidine incorporation (see, e.g., Chen, J., 1996, Oncogene 13:1395-403; Jeoung, J., 1995, J. Biol. Chem. 270:18367-73), by direct cell count, by detecting changes in transcription, translation or activity of known genes such as proto-oncogenes (*e.g.*, *fos*, *myc*) or cell cycle markers (Rb, cdc2, cyclin A, D1, D2, D3, E, etc). The levels of such protein and mRNA and activity can be determined by any method well known in the art. For example, protein can be quantitated by known immunodiagnostic methods such as Western blotting or immunoprecipitation using commercially available antibodies. mRNA can be quantitated using methods that are well known and routine in the art, for example, using northern analysis, RNase protection, the polymerase chain reaction in connection with the reverse transcription. Cell viability can be assessed by using trypan-blue staining or other cell death or viability markers known in the art. In a specific embodiment, the level of cellular ATP is measured to determined cell viability. Differentiation can be assessed, for example, visually based on changes in morphology.

The therapies of the invention (in particular, the combinations of prophylactic and/or therapeutic agents) can be tested in suitable animal model systems prior to use in humans. Such animal model systems include, but are not limited to, rats, mice, chicken, cows, monkeys, pigs, dogs, rabbits, etc. Any animal system well-known in the art may be used. Such model systems are widely used and well-known to the skilled artisan. Prophylactic and/or therapeutic agents can be administered repeatedly. Several aspects of the procedure may vary. Said aspects include the temporal regime of administering the prophylactic and/or therapeutic agents, and whether such agents are administered separately or as an admixture.

An illustrative animal model system for testing antiviral effects of Glycyrrhizin is described in Utsunomiya et al., 1996, Antimicrobial Agents and Chemotherapy 41(3):551-556 (*e.g*., at Materials And Methods), which is incorporated herein in its entirety. The antiviral effect of a compound in an animal model system can be determined based on parameters such as survival rate, virus titer in specimen from the infected animal, pathological effects of the vims-infection on cells, tissues and/or organs in the animal. Controls include animals that were not infected with the virus and/or animals that were not treated with the Glycyrrhizin or derivative thereof.

The therapies (*e.g.*, prophylactic or therapeutic agents) can be assessed for their ability to inhibit or reduce a viral infection (*e.g*., SARS-associated coronavirus infection) *in vivo*. For example, the prophylactic or therapeutic agents can be administered to a test animal, preferably a test animal exposed to a SARS-associated coronavirus, and the test animal subsequently examined for viral titer, antibodies against the virus, proteins or nucleic acids of the virus, or any SARS-related symptoms.

The toxicity and/or efficacy of the prophylactic and/or therapeutic protocols of the instant invention can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀.

The data obtained from the cell culture assays and animal studies can be used in formulating a range of dosage of prophylactic or therapeutic agents for use in humans. The dosage of such agents lies preferably within a range of circulating concentrations that include the ED₅₀ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the IC₅₀ (*i.e.*, the concentration of the test compound that achieves a half-maximal inhibition of symptoms) as determined in cell culture. Such information can be used to more accurately determine useful doses in humans. Levels in plasma may be measured, for example, by high performance liquid chromatography (HPLC) and radioimmunasssay (RIA). The pharmacokinetics of a prophylactic or therapeutic agent can be determined, e.g., by measuring parameters such as peak plasma level (Cₘₐₓ), area under the curve (AUC, which is measured by plotting plasma concentration of the agent versus time, and reflects bioavailability), half-life of the compound (t_{1/2}), and time at maximum concentration.

Further, any assays known to those skilled in the art can be used to evaluate the prophylactic and/or therapeutic utility of the therapies of invention for a virus infection (*e.g*., a SARS-associated coronavirus infection) disclosed herein.

### 5.7. METHODS OF ADMINISTERING

The present invention provides methods for the prevention, treatment, management, and amelioration of a virus infection (*e.g*., SARS-associated coronavirus infection) or one or more symptoms thereof. In a specific embodiment, a composition comprises Glycyrrhizin or a derivative thereof. In another embodiment, a composition comprises Glycyrrhizin or a derivative thereof and one or more prophylactic or therapeutic agents other than Glycyrrhizin or a derivative thereof In another embodiment, a composition comprises Glycyrrhizin or a derivative thereof and one or more antiviral agents. In another embodiment, a composition comprises Glycyrrhizin or a derivative thereof and Ribavirin. In accordance with these embodiments, the composition may further comprise of a carrier.

The compositions of the invention include, but are not limited to, bulk drug compositions useful in the manufacture of pharmaceutical compositions (*e.g*., impure or non-sterile compositions) and pharmaceutical compositions (*i.e.,* compositions that are suitable for administration to a subject or patient) which can be used in the preparation of unit dosage forms. Such compositions comprise a prophylactically or therapeutically effective amount of a prophylactic and/or therapeutic agent disclosed herein or a combination of those agents and a pharmaceutically acceptable carrier. Preferably, compositions of the invention are pharmaceutical compositions and comprise an effective amount of Glycyrrhizin or a derivative thereof, a pharmaceutically acceptable carrier, and, optionally, an effective amount of another prophylactic or therapeutic agent.

In a specific embodiment, the term "pharmaceutically acceptable" means approved by a regulatory agency of the Federal or a state government or listed in the U.S. Pharmacopeia, European Pharmacopeia, or other generally recognized pharmacopeia for use in animals, and more particularly in humans. The term "carrier" refers to a diluent, adjuvant (*e.g*., Freund's adjuvant (complete and incomplete)), excipient, or vehicle with which the therapeutic is contained in or administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compositions of the invention can be formulated as neutral or salt forms. Pharmaceutically acceptable salts include those formed with anions such as those derived from hydrochloric, phosphoric, acetic, oxalic, tartaric, butyric acids, etc., and those formed with cations such as those derived from sodium, potassium, ammonium, calcium, ferric hydroxides, isopropylamine, triethylamine, 2-ethylamino ethanol, histidine, procaine, etc.

Various delivery systems are known and can be used to administer Glycyrrhizin or a derivative thereof or the combination of Glycyrrhizin or a derivative thereof and a prophylactic agent or therapeutic agent other than Glycyrrhizin or a derivative thereof useful for preventing, managing, treating, or ameliorating a respiratory condition (preferably, a viral respiratory infection and most preferably, a SARS-associated coronavirus infection) or one or more symptoms thereof, *e.g*., encapsulation in liposomes, microparticles, microcapsules, recombinant cells capable of expressing the antibody or antibody fragment, receptor-mediated endocytosis (see, *e.g*., Wu and Wu, J. Biol. Chem. 262:4429-4432 (1987)), construction of a nucleic acid as part of a retroviral or other vector, etc. Methods of administering a prophylactic or therapeutic agent of the invention include, but are not limited to, parenteral administration (*e.g*., intradermal, intramuscular, intraperitoneal, intravenous and subcutaneous), epidurala administration, intratumoral administration, and mucosal adminsitration (*e.g*., intranasal and oral routes). In addition, pulmonary administration can be employed, e.g., by use of an inhaler or nebulizer, and formulation with an aerosolizing agent. See, e.g., U.S. Patent Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entirety. In one embodiment, Glycyrrhizin or a derivative thereof, a combination therapy, or a composition of the invention is administered using Alkermes AIR^{™} pulmonary drug delivery technology (Alkermes, Inc., Cambridge, MA). In a specific embodiment, prophylactic or therapeutic agents of the invention are administered intramuscularly, intravenously, intratumorally, orally, intranasally, pulmonary, or subcutaneously. The prophylactic or therapeutic agents may be administered by any convenient route, for example by infusion or bolus injection, by absorption through epithelial or mucocutaneous linings (*e.g*., oral mucosa, rectal and intestinal mucosa, etc.) and may be administered together with other biologically active agents. Administration can be systemic or local.

In a specific embodiment, it may be desirable to administer the prophylactic or therapeutic agents of the invention locally to the area in need of treatment; this may be achieved by, for example, and not by way of limitation, local infusion, by injection, or by means of an implant, said implant being of a porous or non-porous material, including membranes and matrices, such as sialastic membranes, polymers, fibrous matrices (e.g., Tissuel^{®}), or collagen matrices. In one embodiment, an effective amount of Glycyrrhizin or a derivative thereof is administered locally to the affected area to a subject to prevent, treat, manage, and/or ameliorate a SARS-associated coronavirus infection or a symptom thereof. In another embodiment, an effective amount of Glycyrrhizin or a derivative thereof is administered locally to the affected area in combination with an effective amount of one or more therapies (*e.g*., one or more prophylactic or therapeutic agents) other than Glycyrrhizin or a derivative thereof of a subject to prevent, treat, manage, and/or ameliorate a SARS-associated coronavirus or one or more symptoms thereof.

The prophylactic or therapeutic agent can be delivered in a controlled-release or sustained release system. The purpose of controlled-release pharmaceutical products is to maximize the benefits of drug therapy while minimizing the amount of drug employed and the time to cure or control the condition. The advantages of controlled-release formulations include extended activity of the drug, reduced dosage frequency and improved compliance by the subject treated. In addition, controlled-release formulations affect the time required for onset of action and other characteristics such as the blood levels of the drug, thus providing some control over the occurrence of side effects.

Most controlled-release formulations are designed to initially release a pharmaceutical agent in an amount that promptly produces the desired effects and then gradually and continually release other ingredients in amounts sufficient to continue the desired effects over an extended period of time. In order to maintain the continued therapeutic and prophylactic effects, the drug must be released from the dosage form at a rate that will replace the amount of drug being metabolized and excreted from the body. Controlled release of a pharmaceutical agent can be stimulated by various conditions including but not limited to pH, temperature, enzymes, water, or other physiological conditions or compounds.

In one embodiment, a pump may be used to achieve controlled or sustained release (see Langer, *supra*; Sefton, 1987, CRC Crit. Ref. Biomed. Eng. 14:20; Buchwald et al., 1980, Surgery 88:507; Saudek et al., 1989, N. Engl. J. Med. 321:574). In another embodiment, polymeric materials can be used to achieve controlled or sustained release of the therapies of the invention (see *e.g.*, Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Patent No. 5,679,377; U.S. Patent No. 5,916,597; U.S. Patent No. 5,912,015; U.S. Patent No. 5,989,463; U.S. Patent No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In a preferred embodiment, the polymer used in a sustained release formulation is inert, free of leachable impurities, stable on storage, sterile, and biodegradable. In yet another embodiment, a controlled or sustained release system can be placed in proximity of the prophylactic or therapeutic target, thus requiring only a fraction of the systemic dose (see, *e.g.*, Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more therapeutic agents of the invention. See, e.g., U.S. Patent No. 4,526,938, PCT publication WO 91/05548, PCT publication WO 96/20698,.Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'1. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760, each of which is incorporated herein by reference in their entirety.

In a specific embodiment, where the composition of the invention is a nucleic acid encoding a prophylactic or therapeutic agent, the nucleic acid can be administered *in vivo* to promote expression of its encoded prophylactic or therapeutic agent, by constructing it as part of an appropriate nucleic acid expression vector and administering it so that it becomes intracellular, e.g., by use of a retroviral vector (see U.S. Patent No. 4,980,286), or by direct injection, or by use of microparticle bombardment (*e.g*., a gene gun; Biolistic, Dupont), or coating with lipids or cell-surface receptors or transfecting agents, or by administering it in linkage to a homeobox-like peptide which is known to enter the nucleus (see, *e.g*., Joliot et al., 1991, Proc. Natl. Acad. Sci. USA 88:1864-1868). Alternatively, a nucleic acid can be introduced intracellularly and incorporated within host cell DNA for expression by homologous recombination.

A pharmaceutical composition of the invention is formulated to be compatible with its intended route of administration. Examples of routes of administration include, but are not limited to, parenteral, *e.g*., intravenous, intradermal, subcutaneous, oral, intranasal (*e.g*., inhalation), transdermal (*e.g*., topical), transmucosal, and rectal administration. In a specific embodiment, the composition is formulated in accordance with routine procedures as a pharmaceutical composition adapted for intravenous, subcutaneous, intramuscular, oral, intranasal, or topical administration to human beings. Typically, compositions for intravenous administration are solutions in sterile isotonic aqueous buffer. Where necessary, the composition may also include a solubilizing agent and a local anesthetic such as lignocamne to ease pain at the site of the injection.

If the compositions of the invention are to be administered topically, the compositions can be formulated in the form of an ointment, cream, transdermal patch, lotion, gel, shampoo, spray, aerosol, solution, emulsion, or other form well-known to one of skill in the art. See, *e.g*., Remington's Pharmaceutical Sciences and Introduction to Pharmaceutical Dosage Forms, 19th ed., Mack Pub. Co., Easton, PA (1995). For non-sprayable topical dosage forms, viscous to semi-solid or solid forms comprising a carrier or one or more excipients compatible with topical application and having a dynamic viscosity preferably greater than water are typically employed. Suitable formulations include, without limitation, solutions, suspensions, emulsions, creams, ointments, powders, liniments, salves, and the like, which are, if desired, sterilized or mixed with auxiliary agents (*e.g*., preservatives, stabilizers, wetting agents, buffers, or salts) for influencing various properties, such as, for example, osmotic pressure. Other suitable topical dosage forms include sprayable aerosol preparations wherein the active ingredient, preferably in combination with a solid or liquid inert carrier, is packaged in a mixture with a pressurized volatile (*e.g*., a gaseous propellant, such as freon) or in a squeeze bottle. Moisturizers or humectants can also be added to pharmaceutical compositions and dosage forms if desired. Examples of such additional ingredients are well-known in the art.

If the method of the invention comprises intranasal administration of a composition, the composition can be formulated in an aerosol form, spray, mist or in the form of drops. In particular, prophylactic or therapeutic agents for use according to the present invention can be conveniently delivered in the form of an aerosol spray presentation from pressurized packs or a nebuliser, with the use of a suitable propellant (*e.g*., dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, *e.g*., gelatin) for use in an inhaler or insufflator may be formulated containing a powder mix of the compound and a suitable powder base such as lactose or starch.

If the method of the invention comprises oral administration, compositions can be formulated orally in the form of tablets, capsules, cachets, gelcaps, solutions, suspensions, and the like. Tablets or capsules can be prepared by conventional means with pharmaceutically acceptable excipients such as binding agents (*e.g*., pregelatinised maize starch, polyvinylpyrrolidone, or hydroxypropyl methylcellulose); fillers (*e.g*., lactose, microcrystalline cellulose, or calcium hydrogen phosphate); lubricants (*e.g*., magnesium stearate, talc, or silica); disintegrants (*e.g*., potato starch or sodium starch glycolate); or wetting agents (*e.g*., sodium lauryl sulphate). The tablets may be coated by methods well-known in the art. Liquid preparations for oral administration may take the form of, but not limited to, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives, or hydrogenated edible fats); emulsifying agents (*e.g*., lecithin or acacia); non-aqueous vehicles (*e.g*., almond oil, oily esters, ethyl alcohol, or fractionated vegetable oils); and preservatives (*e.g*., methyl or propyl-p-hydroxybenzoates or sorbic acid). The preparations may also contain buffer salts, flavoring, coloring, and sweetening agents as appropriate. Preparations for oral administration may be suitably formulated for slow release, controlled release, or sustained release of a prophylactic or therapeutic agent(s).

The method of the invention may comprise pulmonary administration, e.g., by use of an inhaler or nebulizer, of a composition formulated with an aerosolizing agent. See, e.g., U.S. Patent Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entirety. In a specific embodiment, Glycyrrhizin or a derivative thereof, combination therapy, and/or composition of the invention is administered using Alkermes AIR^{™} pulmonary drug delivery technology (Alkermes, Inc., Cambridge, MA).

The method of the invention may comprise administration of a composition formulated for parenteral administration by injection (*e.g*., by bolus injection or continuous infusion). Formulations for injection may be presented in unit dosage form (*e.g*., in ampoules or in multi-dose containers) with an added preservative. -The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle (*e.g*., sterile pyrogen-free water) before use.

Suitable vehicles that can be used to provide parenteral dosage forms of the invention are well-known to those skilled in the art. In certain embodiments, suitable vehicles for parenteral dosage forms include but are not limited to Water for Injection USP; aqueous vehicles including but not limited to Sodium Chloride Injection, Ringer's Injection, Dextrose Injection, Dextrose and Sodium Chloride Injection and Lactated Ringer's Injection; water-miscible vehicles including but not limited to ethyl alcohol, polyethylene glycol and polypropylene glycol; and non-aqueous vehicles including but not limited to corn oil, cottonseed oil, peanut oil, sesame oil, ethyl oleate, isopropyl myristate and benzyl benzoate.

In other embodiments, compounds that increase the solubility of the prophylactic or therapeutic agents are incorporated into the parenteral dosage forms. For example, cyclodextrin and its derivatives can be used to increase the solubility of a thalidomide analogue and its derivatives. *See*, *e.g.*, U.S. Patent No. 5,134,127, which is incorporated herein by reference. The methods of the invention may additionally comprise of administration of compositions formulated as depot preparations. Such long acting formulations may be administered by implantation (*e.g*., subcutaneously or intramuscularly) or by intramuscular injection. Thus, for example, the compositions may be formulated with suitable polymeric or hydrophobic materials (*e.g*., as an emulsion in an acceptable oil) or ion exchange resins, or as sparingly soluble derivatives (*e.g*., as a sparingly soluble salt).

The methods of the invention encompasses administration of compositions formulated as neutral or salt forms. Pharmaceutically acceptable salts include those formed with anions such as those derived from hydrochloric, phosphoric, acetic, oxalic, tartaric, butyric acids, etc., and those formed with cations such as those derived from sodium, potassium, ammonium, calcium, ferric hydroxides, isopropylamine, triethylamine, 2-ethylamino ethanol, histidine, procaine, etc.

Generally, the ingredients of compositions are supplied either separately or mixed together in unit dosage form, for example, as a dry lyophilized powder or water free concentrate in a hermetically sealed container such as an ampoule or sachette indicating the quantity of active agent. Where the mode of administration is infusion, composition can be dispensed with an infusion bottle containing sterile pharmaceutical grade water or saline. Where the mode of administration is by injection, an ampoule of sterile water for injection or saline can be provided so that the ingredients may be mixed prior to administration.

In particular, the invention also provides that one or more of the prophylactic or therapeutic agents, or pharmaceutical compositions of the invention is packaged in a hermetically sealed container such as an ampoule or sachette indicating the quantity of the agent. In one embodiment, one or more of the prophylactic or therapeutic agents, or pharmaceutical compositions of the invention is supplied as a dry sterilized lyophilized powder or water free concentrate in a hermetically sealed container and can be reconstituted (*e.g*., with water or saline) to the appropriate concentration for administration to a subject.

The compositions may, if desired, be presented in a pack or dispenser device that may contain one or more unit dosage forms containing the active ingredient. The pack may for example comprise metal or plastic foil, such as a blister pack. The pack or dispenser device may be accompanied by instructions for administration.

Generally, the ingredients of the compositions of the invention are derived from a subject that is the same species origin or species reactivity as recipient of such compositions. Thus, in a preferred embodiment, human or humanized antibodies are administered to a human patient for therapy or prophylaxis.

### 5.8. DOSAGE AND FREQUENCY OF ADMINISTRATION

The prophylactically or therapeutically effective amount of Glycyrrhizin or a derivative thereof which will be effective in the prevention, treatment, management or amelioration of a virus infection (*e.g*., SARS-associated coronavirus infection) or one or more symptoms thereof can be determined by standard clinical techniques. The dose, dose frequency, or both, will depend on the age of the patient, the patient's body weight, the patient's response, the seriousness of the patient's condition, and the past medical history of the patient as well as the route of administration, pharmacokinetic and pharmacodynamic effects of the prophylactic or therapeutic agent, and should be decided according to the judgment of the practitioner and each patient's circumstances. Effective doses may be extrapolated from dose-response curves derived from *in vitro* or animal model test systems (see Section 5.6, *infra*).

Exemplary doses of Glycyrrhizin or a derivative thereof include milligram or microgram amounts of Glycyrrhizin or a derivative thereof per kilogram of subject or sample weight (*e.g*., about 1 microgram per kilogram to about 500 milligrams per kilogram, about 100 micrograms per kilogram to about 5 milligrams per kilogram, or about 1 microgram per kilogram to about 50 micrograms per kilogram). In specific embodiments, a daily dose of Glycyrrhizin or a derivative thereof is at least 50 mg, 75 mg, 100 mg, 150 mg, 250 mg, 500 mg, 750 mg, or at least 1 g.

In one embodiment, the dosage of a Glycyrrhizin or a derivative thereof or a composition comprising Glycyrrhizin or a derivative thereof for use in the prevention, treatment, management or amelioration of a viral infection or one or more symptoms thereof is a concentration of 0.01 to 5000 mM, 1 to 300 mM, 10 to 100 mM and 10 mM to 1 M. In another embodiment, the dosage of a Glycyrrhizin or a derivative thereof or a composition comprising a Glycyrrhizin or a derivative thereof for use in the prevention, treatment, management or amelioration of a viral infection or one or more symptoms thereof is a concentration of at least 5 µM, at least 10 µM, at least 50 µM, at least 100 µM, at least 500 µM, at least 1 mM, at least 5 mM, at least 10 mM, at least 50 mM, at least 100 mM, or at least 500 mM.

In one embodiment, the dosage of a Glycyrrhizin or a derivative thereof or a composition comprising Glycyrrhizin or a derivative thereof for use in the prevention, treatment, management or amelioration of a SARS-associated coronavirus infection or one or more symptoms thereof is a concentration of 0.01 to 5000 mM, 1 to 300 mM, 10 to 100 mM and 10 mM to 1 M. In another embodiment, the dosage of a Glycyrrhizin or a derivative thereof or a composition comprising a Glycyrrhizin or a derivative thereof for use in the prevention, treatment, management or amelioration of a SARS-associated coronavirus infection or one or more symptoms thereof is a concentration of at least 5 µM, at least 10 µM, at least 50 µM, at least 100 µM, at least 500 µM, at least 1 mM, at least 5 mM, at least 10 mM,at least 50 mM, at least 100 mM, or at least 500 mM.

In a specific embodiment, the dosage of a Glycyrrhizin or a derivative thereof or a composition comprising Glycyrrhizin or a derivative thereof for use in the prevention, treatment, management or amelioration of a SARS-associated coronavirus infection or one or more symptoms thereof in a patient is 0.25 µg/kg or more, preferably 0.5 µg/kg or more, 1 µg/kg or more, 2 µg/kg or more, 3 µg/kg or more, 4 µg/kg or more, 5 µg/kg or more, 6 µg/kg or more, 7 µg/kg or more, 8 µg/kg or more, 9 µg/kg or more, or 10 µg/kg or more, 25 µg/kg or more, preferably 50 µg/kg or more, 100 µg/kg or more, 250 µg/kg or more, 500 µg/kg or more, 1 mg/kg or more, 5 mg/kg or more, 6 mg/kg or more, 7 mg/kg or more, 8 mg/kg or more, 9 mg/kg or more, or 10 mg/kg or more of a patient's body weight. In another embodiment, the dosage of a Glycyrrhizin or a derivative thereof or a composition comprising a Glycyrrhizin or a derivative thereof for use in the prevention, treatment, management or amelioration of a SARS-associated coronavirus infection or one or more symptoms thereof in a patient is a unit dose of 5 mg, preferably 10 mg, 50 mg, 100 mg, 150 mg, 200 mg, 250 mg, 300 mg, 350 mg, 400 mg, 500 mg, 550 mg, 600 mg, 650 mg, 700 mg, 750 mg, 800 mg or more. In another embodiment, the dosage of a Glycyrrhizin or a derivative thereof or a composition comprising a Glycyrrhizin or a derivative thereof for use in the prevention, treatment, management or amelioration a SARS-associated coronavirus infection or one or more symptoms thereof in a patient is a unit dose that ranges from about 5 mg to about 100 mg, preferably about 100 mg to about 200 µg, about 150 mg to about 300 mg, about 150 mg to about 400 mg, 250 µg to about 500 mg, about 500 mg to about 800 mg, about 500 mg to about 1000 mg, or about 5 mg to about 1000 mg.

The dosages of prophylactic or therapeutic agents other than Glycyrrhizin or a derivative thereof which have been or are currently being used for the prevention, treatment or amelioration of a viral infection (preferably, a SARS-associated coronavirus infection) or a symptom thereof can be determined using references available to a clinician such as, e.g., the Physicians' Desk Reference (55th ed. 2001). Preferably, dosages lower than those which have been or are currently being used to prevent, treat or ameliorate a viral infection (preferably, a SARS-associated coronavirus infection) are utilized in combination with a Glycyrrhizin or a derivative thereof.

In another embodiment, a subject is administered one or more doses of a prophylactically or therapeutically effective amount of a Glycyrrhizin or a derivative thereof, wherein the prophylactically or therapeutically effective amount is not the same for each dose. In another embodiment, a subject is administered one or more doses of a prophylactically or therapeutically effective amount of Glycyrrhizin or a derivative thereof, wherein the dose of a prophylactically or therapeutically effective amount of the Glycyrrhizin or a derivative thereof administered to said subject is increased by, e.g., 0.01 µg/kg, 0.02 µg/kg, 0.04 µg/kg, 0.05 µg/kg, 0.06 µg/kg, 0.08 µg/kg, 0.1 µg/kg, 0.2 µg/kg, 0.25 µg/kg, 0.5 µg/kg, 0.75 µg/kg, 1 µg/kg, 1.5 µg/kg, 2 µg/kg, 4 µg/kg, 5 µg/kg, 10 µg/kg, 15 µg/kg, 20 µg/kg, 25 µg/kg, 30 µg/kg, 35 µg/kg, 40 µg/kg, 45 µg/kg, or 50 µg/kg, as treatment progresses. In another embodiment, a subject is administered one or more doses of a prophylactically or therapeutically effective amount of Glycyrrhizin or a derivative thereof, wherein the dose of a prophylactically or therapeutically effective amount of the Glycyrrhizin or a derivative thereof administered to said subject is decreased by, e.g., 0.01 µg/kg, 0.02 µg/kg, 0.04 µg/kg, 0.05 µg/kg, 0.06 µg/kg, 0.08 µg/kg, 0.1 µg/kg, 0.2 µg/kg, 0.25 µg/kg, 0.5 µg/kg, 0.75 µg/kg, 1 µg/kg, 1.5 µg/kg, 2 µg/kg, 4 µg/kg, 5 µg/kg, 10 µg/kg, 15 µg/kg, 20 µg/kg, 25 µg/kg, 30 µg/kg, 35 µg/kg, 40 µg/kg, 45 µg/kg, or 50 µg/kg, as treatment progresses.

In certain embodiments, a subject is administered one or more doses of an effective amount of Glycyrrhizin or a derivative thereof, wherein the dose of an effective amount of said Glycyrrhizin or derivative thereof inhibits or reduces the replication of a SARS-associated coronavirus by at least 20% to 25%, preferably at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, or up to at least 85%. In other embodiments, a subject is administered one or more doses of an effective amount of Glycyrrhizin or a derivative thereof, wherein the dose of an effective amount of said Glycyrrhizin or derivative thereof inhibits or reduces the production of SARS-associated coronavirus particles by at least 20% to 25%, preferably at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, or up to at least 85%. In other embodiments, a subject is administered one or more doses of an effective amount of Glycyrrhizin or a derivative thereof, wherein the dose of an effective amount of said Glycyrrhizin or derivative thereof inhibits or reduces the release of SARS-associated coronavirus particles by at least 20% to 25%, preferably at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, or up to at least 85%.

In a specific embodiment, the invention provides methods of preventing, treating, managing or ameliorating a SARS-associated coronavirus infection, said method comprising administering to a subject in need thereof a dose of at least 5 mg, preferably 10 mg, 50 mg, 100 mg, 150 mg, 200 mg, 250 mg, 300 mg, 350 mg, 400 mg, 500 mg, 550 mg, 600 mg, 650 mg, 700 mg, 750 mg, 800 mg or more of Glycyrrhizin or a derivative thereof once every day, once every 2 days, once every 3 days, once every 4 days, once every 5 days, once every 6 days, once every 7 days, once every 8 days, once every 10 days, once every two weeks, or once every three weeks for a certain period of time.

The above-described administration schedules are provided for illustrative purposes only and should not be considered limiting. A person of ordinary skill in the art will readily understand that all doses of Glycyrrhizin or a derivative thereof are within the scope of the invention.

Therapies (*e.g*., prophylactic or therapeutic agents), other than Glycyrrhizin or a derivative thereof, which have been or are currently being used to prevent, treat, manage, or ameliorate a condition (preferably, a viral condition) or one or more symptoms thereof can be administered in combination with Glycyrrhizin or a derivative thereof according to the methods of the invention to treat, manage, prevent, or ameliorate a viral infection or one or more symptoms thereof.

Therapies (*e.g*., prophylactic or therapeutic agents), other than Glycyrrhizin or a derivative thereof, which have been or are currently being used to prevent, treat, manage, or ameliorate a respiratory condition (preferably, a viral respiratory condition and most preferably, a SARS-associated coronavirus infection) or one or more symptoms thereof can be administered in combination with Glycyrrhizin or a derivative thereof according to the methods of the invention to treat, manage, prevent, or ameliorate a SARS-associated coronavirus infection or one or more symptoms thereof. Preferably, the dosages of prophylactic or therapeutic agents used in combination therapies of the invention are lower than those which have been or are currently being used to prevent, treat, manage, or ameliorate a respiratory condition (preferably, a viral respiratory condition and most preferably, a SARS-associated coronavirus infection) or one or more symptoms thereof. The recommended dosages of agents currently used for the prevention, treatment, management, or amelioration of a condition (preferably, a viral respiratory condition) or one or more symptoms thereof can be obtained from any reference in the art including, but not limited to, Hardman et al., eds., 2001, Goodman & Gilman's The Pharmacological Basis Of Basis Of Therapeutics, 10th ed., Mc-Graw-Hill, New York; Physician's Desk Reference (PDR) 57th ed., 2003, Medical Economics Co., Inc., Montvale, NJ, which are incorporated herein by reference in its entirety.

In various embodiments, the therapies (*e.g*., prophylactic or therapeutic agents) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In preferred embodiments, two or more therapies are administered within the same patent visit.

In certain embodiments, Glycyrrhizin or a derivative thereof and one or more other therapies (*e.g*., prophylactic or therapeutic agents) are cyclically administered. Cycling therapy involves the administration of a first therapy (*e.g*., a first prophylactic or therapeutic agent) for a period of time, followed by the administration of a second therapy (*e.g*., a second prophylactic or therapeutic agent) for a period of time, optionally, followed by the administration of a third therapy (*e.g.*, prophylactic or therapeutic agent) for a period of time and so forth, and repeating this sequential administration, *i.e*., the cycle in order to reduce the development of resistance to one of the therapies, to avoid or reduce the side effects of one of the therapies, and/or to improve the efficacy of the therapies.

In certain embodiments, the administration of the same Glycyrrhizin derivative may be repeated and the administrations may be separated by at least 1 day, 2 days, 3 days, 5 days, 10 days, 15 days, or 30 days. In other embodiments, the administration of the same therapy (e.g., prophylactic or therapeutic agent) other than Glycyrrhizin or a derivative thereof may be repeated and the administration may be separated by at least at least 1 day, 2 days, 3 days, 5 days, 10 days, 15 days, or 30 days.

### 5.9. ARTICLES OF MANUFACTURE

The present invention also encompasses a finished packaged and labeled pharmaceutical product. This article of manufacture includes the appropriate unit dosage form in an appropriate vessel or container such as a glass vial or other container that is hermetically sealed. In the case of dosage forms suitable for parenteral administration the active ingredient, e.g., Glycyrrhizin or a derivative thereof, is sterile and suitable for administration as a particulate free solution. In other words, the invention encompasses both parenteral solutions and lyophilized powders, each being sterile, and the latter being suitable for reconstitution prior to injection. Alternatively, the unit dosage form may be a solid suitable for oral, transdermal, intransal, pulmonary or topical delivery.

In certain embodiments, the unit dosage form is suitable for intravenous, intramuscular, intranasal, oral, topical, pulmonary, or subcutaneous delivery. Thus, the invention encompasses solutions, preferably sterile, suitable for each delivery route.

As with any pharmaceutical product, the packaging material and container are designed to protect the stability of the product during storage and shipment. Further, the products of the invention include instructions for use or other informational material that advise the physician, technician or patient on how to appropriately prevent or treat the respiratory condition in question. In other words, the article of manufacture includes instruction means indicating or suggesting a dosing regimen and monitoring information including, but not limited to, actual doses, monitoring procedures, total lymphocyte counts, mast cell counts, mast cell degranulation, red blood cell counts, T cell counts, IgE antibody production, and other monitoring information.

Specifically, the invention provides an article of manufacture comprising packaging material, such as a box, bottle, tube, vial, container, sprayer, insufflator, intravenous (i.v.) bag, envelope and the like; and at least one unit dosage form of a pharmaceutical agent contained within said packaging material, wherein said pharmaceutical agent comprises Glycyrrhizin or a derivative thereof and wherein said packaging material includes instruction means which indicate that said Glycyrrhizin or derivative thereof can be used to treat, prevent, manage, or ameliorate a SARS-associated coronavirus infection or one or more symptoms thereof by administering specific doses and using specific dosing regimens as described herein.

The invention also provides an article of manufacture comprising packaging material, such as a box, bottle, tube, vial, container, sprayer, insufflator, intravenous (i.v.) bag, envelope and the like; and at least one unit dosage form of each pharmaceutical agent contained within said packaging material, wherein one pharmaceutical agent comprises Glycynhizin or a derivative thereof and a second pharmaceutical agent comprises a prophylactic or therapeutic agent, other than Glycyrrhizin or a derivative thereof, and wherein said packaging material includes instruction means which indicate that said Glycyrrhizin or derivative thereof can be used to treat, prevent, manage, or ameliorate a SARS-associated coronavirus infection or one or more symptoms thereof by administering specific doses and using specific dosing regimens as described herein.

The invention also provides an article of manufacture comprising packaging material, such as a box, bottle, tube, vial, container, sprayer, insufflator, intravenous (i.v.) bag, envelope and the like; and at least one unit dosage form of a pharmaceutical agent contained within said packaging material, wherein one pharmaceutical agent comprises Glycyrrhizin or a derivative thereof and a prophylactic or therapeutic agent other than Glycyrrhizin or a derivative thereof and wherein said packaging material includes instruction means which indicate that said agents can be used to treat, prevent, manage, or ameliorate a SARS-associated coronavirus infection or one or more symptoms thereof by administering specific doses and using specific dosing regimens as described herein.

The present invention provides that the adverse effects that may be reduced or avoided by the methods of the invention are indicated in informational material enclosed in an article of manufacture for use in preventing, treating, managing, or ameliorating a SARS-associated coronavirus infection or one or more symptoms thereof. Adverse effects that may be reduced or avoided by the methods of the invention include, but are not limited to, vital sign abnormalities (fever, tachycardia, bardycardia, hypertension, hypotension), hematological events (anemia, lymphopenia, leukopenia, thrombocytopenia), headache, chills, dizziness, nausea, asthenia, back pain, chest pain (chest pressure), diarrhea, myalgia, pain, pruritus, psoriasis, rhinitis, sweating, injection site reaction, and vasodilatation.

### 6.1. EXAMPLE 1: GLYCYRRHIZIN, AN ACTIVE COMPONENT OF LIQUORICE ROOTS, AND REPLICATION OF SARS-ASSOCIATED CORONAVIRUS

The outbreak of SARS warrants the research for antiviral compounds to treat disease. At present, no specific treatment has been identified for a SARS-associated coronavirus infection. The antiviral potential of ribavirin, 6-azauridine, pyrazofurin, mycophenolic acid, and Glycyrrhizin against two clinical isolates of coronavirus (FFM-1 and FFM-2) from patients with SARS admitted to the clinical centre of the University of Frankfurt, Germany, were assessed. All the compounds are commercially available and have been used in patients for their antiviral, antitumour, and immunosuppressive activity. Of all the compounds, Glycyrrhizin was the most active in inhibiting replication of the SARS-associated coronavirus. These result indicate the prophylactic and therapeutic utility of Glycyrrhizin.

Cytopathogenicity induced by the virus 72-96 h after infection in 96-well microplates on confluent layers of Vero cells was visually scored. The selectivity index was determined as the ratio of the concentration of the compound that reduced cell viability to 50% (CC₅₀) to the concentration of the compound needed to inhibit the cytopathic effect to 50% of contral value (EC₅₀). The cytotoxicity of the drug with an MMT cell-proliferative Kit I (Roche, Mannheim, Germany) was determined.

Ribavirin and mycophoenolic acid, inhibitors of inosine monophosphae dehydrogenase, did not affect replication of the SARS-associated coronaviruses (SARS-CV) (see Table 2). The inhibitors of orotidine monophosphate decarboxylase, 6-azuridine and pyrazofurin, inhibited replication of SARS-CV at non-toxic doses with selectivity indices of 5 and 12, respectively. The most potent inhibitor of SARS-CV replication in Vero cells was Glycyrrhizin, which had a selectivity index of 67.

**TABLE 2: Activity of compounds against SARS-Associated coronavirus in Vero cell cultures**

| | EC₅₀* (mg/L) | CC₅₀* (mg/L) | Selectivity Index |
|---|---|---|---|
| Compound | | | |
| 6-azauridine | 16-8 | 104 | 6 |
| Pyrazofurin | 4-2 | 52 | 12 |
| Mycophenolic acid | >50 | >50 | NC |
| Ribavirin | >1000 | >1000 | NC |
| Glycyrrhizin | | | |
| After virus adsorption | 600 | >20,000† | >33 |
| During and after virus adsorption | 300 | >20,000 | >67 |
| During virus adsorption | 2400 | >20,000 | >8-3 |

| | | | |
|---|---|---|---|
| EC₅₀= effective concentration of compound needed to inhibit the cytopathic effect to 50% of control value. CC₅₀=cytotoxic concentration of the compound that reduced cell viability to 50%. NC=Not Calculable. * Mean (SD) of eight assays. † At the maximum concentration used (200, mg/L) a 20-30% reduction of cell viability was recorded. | | | |

In addition to inhibiting virus replication, Glycyrrhizin inhibits adsorption and penetration of the virus-early steps of the replicative cycle. Glycyrrhizin was less effective when added during the adsorption period than when added after virus adsorption (EC₅₀ 600 *vs* 2400 mg/L, respectively). Glycyrrhizin was most effective when given both during and after the adsorption period (EC₅₀ 300 mg/L).

Figure 1 shows the effect of Glycyrrhizin on replication of SARS-CV in Vero cells. Replication of SARS-CV using serum samples from patients with SARS was determined. Expression of viral antigens was much lower in cultures expression of viral antigens was much lower in cultures treated with 1000 mg/L of Glycyrrhizin than in any other culture; high concentrations of Glycyrrhizin (4000 mg/L) completely blocked replication of the virus (Figure 1).

Glycyrrhizin affects cellular signaling pathways such as protein kinase C; casein kinase II; and transcription factors such as activator protein 1 and nuclear factor kappaB. Furthermore, Glycyrrhizin and its aglycone metabolite 18β- Glycyrrhizinic acid upregulate expression of inducible nitrous oxide synthase and production of nitrous oxide in macrophases ((Jeong HG, Kim JY. Induction of inducible nitric oxide synthase expression by 18β-glycyrrhetinic acide in macrophases. FEBS Lett 2002; 513: 208-12). Nitrous oxide inhibits replication of several viruses e.g., Japanese encephalitis virus (a member of the Flavivirate family), which can also be inhibited by Glycyrrhizin. Without being bound by theory, Glycyrrhizin induces nitrous oxide synthase and the nitrous oxide synthase inhibits SARS-associated coronavirus replication. In results not shown here, Glycyrrhizin induced nitrous oxide synthase in Vero cells and SARS-associated coronavirus replication was inhibited when nitrous oxide donor (DETANONOate) was added to the culture medium.

### 6.2 EXAMPLE 2: ANTI-SARS ACTIVITY OF GLYCYRRHIZIN DERIVATIVES

### SYNTHESIS OF GLYCYRRHIZIN DERIVATIVES

Amino acid derivatives of Glycyrrhizin (e.g., Compound 6 and Compound 3) were synthesized by using activated N-hydroxysuccinimide esters as reported in L.A.Baltina et al. Transformations of Glycyrrhizic Acid. VIII. Synthesis of Immunomodulating Glycopeptides using tert-Butyl Esters of Amino Acids; Russian J. Bioorg. Chem., 1994, 20 (12), 778-784; Baltina L.A. et al., Transformations of Glycyrrhizic Acid. VII. Synthesis of Triterpene Glycopeptides containing Alkyl Esters of L-Amino Acids, Khim. Prir. Soedin., 1994, (20), 261-268; and R.M.Kondratenko, et al., Synthesis and Immunostimulating Activity of Cysteine-Containing Derivatives of Glycyrrhizic Acid, Russian J. Bioorg. Chem., 2004, 30 (1),61-67.

The selective synthesis of Compound 3 was carried out by using t-butyl esters of Gly-L-Val hydrochloride as amino component in tetrahydrofuran at 0°C in the presence of N,N'-dicyclohexylcarbodiiimide (DCC) - N-hydroxysuccinimide and a small excess (1 mmol) of a base (triethylamine). T-Butyl ester groups were debloked with CF₃COOH and the target products with free 30-COOH function were isolated by column chromatography (CC) in yields of 50-53%.

The selective synthesis of an amino acid derivative of Glycyrrhizin containing S-benzyl-L-cysteine at R₁ and R₂ was carried out by using t-butyl esters of L-Cys(SBn) hydrochloride as amino components in tetrahydrofuran at 0°C in the presence of N,N'-dicyclohexylcarbodiiimide (DCC) - N-hydroxysuccinimide and a small excess (1 mmol) of a base (triethylamine). T-Butyl ester groups were debloked with CF₃COOH and the target products with free 30-COOH function were isolated by column chromatography (CC) in yields of 50-53%.

Comound **7** was produced by the reaction of Glycyrrhizin trimethyl ester (L.A.Baltina, et al., Transformations of Glycyrrhizic Acid. X. Synthesis of New Esters, Zh. Org. Khim., 1994, 30 (11), 1622-1626) with hydrazine hydrate in methanol at boiling with the yield of 77%.

Heterocyclic amides Compound 2 and Compound 4 were prepared by the reaction of Glycyrrhizin with 5-aminouracyl and 6-amino-2-thio-uracyl the presence of DCC.

β-D-Glucopyranosyl-(1→2)-β-D-Glucopyranoside of 18β-Glycyrrhetinic Acid methyl ester was synthesized by the reduction of Glycyrrhizin trimethyl ester in methanol with NaBH₄ under mild conditions as described in Baltina L.A. et al., Transformations of Glycyrrhizic acid: The Synthesis of 3-O-[β-6'-Deoxy-6'-Amino-D-Glucopyranosyl(1-2)-β-6"Deoxy-6"-Amino-D-Glucopyranosido]-(3β,20β)-11-Oxo-20-Methoxycarbonyl-18β-olean-12-en-3-ol, Mendeleev Comm., 1995, (5β), 178-179.

Comound **5** was synthesized by coupling of Gycyrrhizin and N-acetyl-β-D-glycopyranosylamine in DMF-pyridine mixture by means of DCC under mild conditions. The product Compound 5 was obtained in homogeneous (TLC) state by column chromatography CC with 42% yield. The yield of Compound 5 was higher (60%) when DCC and N-hydroxybenzotriazol were used for the coupling reaction (R.M.Kondratenko et al. Russian J. Bioorg. Chem., 2004, 30 (3), 1-8).

Structures of compounds synthesized were confirmed by IR, UV, NMR ¹H and ¹³C spectra.

The activity of different Glycyrrhizin derivatives against SARS-Associated coronavirus in Vero cell cultures was tested as described in Example 1 above. The activities of the different Glycyrrhizin derivatives is shown in Table 3 below.

**TABLE 3: Activity of compounds against SARS-Associated coronavirus in Vero cell cultures**

| Derivat | EC₅₀µM | CC₅₀µM | Therap. IndexI |
|---|---|---|---|
| Compound **6** | 35 | 1462 | 41 |
| Compound **7** | 16 | 66 | 4 |
| Compound **8** | 8 | 44 | 6 |
| Compound **2** | 5 | 15 | 3 |
| Glycyrrhizin Monoammonium Salt | 327 | 625 | 2 |
| Compound **3** | 139 | 250 | 2 |
| Compound **4** | 50 | 250 | 5 |
| Compound **5** | 47 | >2000 | >43 |

### 6.3 EXAMPLE 3: SYNERGISTIC EFFECT OF THE COMBINATION OF GLYCYRRHICIN AND RIBAVIRIN ON SARS ASSOCIATED CORONAVIRUS

To test the effect of the combination of Glycyrhizin and Ribavirin on SARS associated coronavirus, Vero cells were infected with SARS-associated coronavirus B and treated with Glycyrhizin, Ribavirin, and a combination of Glycyrhizin and Ribavirin, respectively. SARS-associated coronavirus was obtained from specimen of a patient infected with SARS-associated coronavirus as described in Drosten et al., 2003, New England Journal of Medicine 348(20):1967-1976, which is incorporated herein by reference in its entirety. Untreated cells were used as control. Subsequently, cells were fixed with 60 parts methanol and 40 parts acetone at 72 hours after infection. Virus was detected in serum from the patient with SARS by peroxidase staining. The results are shown in Figure 2. Figure 2(A) shows infected cells without treatment; Figure 2(B) shows infected cells treated with 50mg/L Ribavirin; Figure 2(C) shows infected cells treated with 100 mg/L Glycyrrhizin; and Figure 2(D) shows infected cells treated with 50 mg/L Ribavirin and 100 mg/L Glycyrrhizin.

The synergistic effect between Ribavirin and Glycyrrhizin allows to use lower concentrations of both compounds. The use of Ribavirin at lower concentrations is particularly beneficial because Ribavirin is known to be toxic at higher concentrations.

### 6.4 EXAMPLE 4: ANTI-FELINE INFECTIOUS PERITONITIS VIRUS AND ANTI-TRANSMISSIBLE GASTROENTERITIS VIRUS ACTIVITY OF GLYCYRRHIZIN DERIVATIVES

### INTRODUCTION

Coronaviruses and arteriviruses infect multiple species of mammals, including humans, causing diseases that range from encephalitis to enteritis (Perlman S., Pathogenesis of coronavirus-induced infections. Review of pathological and immunological aspects. Adv Exp Med Biol. 1998;440:503-13). Several of these viruses infect domestic animals and cause significant morbidity and mortality, leading to major economic losses. In this category are included such pathogens as transmissible gastroenteritis virus (TGEV), porcine respiratory and reproductive virus and infectious bronchitis virus. The feline coronaviruses (FECV) generally do not cause infections with high morbidity but in a small percentage of cases, the virus mutates to become more virulent. Feline infectious peritonitis virus (FIPV), causes severe disease in young cats. This disease is in large part immunopathological and understanding it is a major goal of coronavirus research.

### MATERIALS AND METHODS

All Coronaviruses and the feline kidney cell line were kindly provided by the Dr. Unger from the Veterinary University of Vienna. 1x10⁶ CRFK cells (ATCC CCL-94) are seeded into a 96 well plate with MEM (+10% FCS, +1% non essential AS, +AB). After 24 hours when confluent the cells are infected with 50 µl FipV (Feline infectious peritonitis Virus) and TGEV (Transmissible gastroenteritis Virus) in MEM without FCS. The cells are incubated for 30min at room temperature before applying Gycyrrhizin derivatives.

Uninfected cells as well as cells treated with control substances are added as controls. The substances are diluted in a 96 well plate in different concentrations beginning with 1000µM and 50µl are transferred to the assay plate. The cells are incubated over night at 37°C. After 48 hours the CPE (Cytopathic effect) is determined by microscopic observation. The proliferative activity of the cells is measured using a standard assay, e.g., CellTiter 96® -AQueous Proliferationassay (Promega).

### RESULTS

The EC₅₀ values for Glycyrrhizin Monoammonium Salt and the Glycyrrhizin derivative Compound **5** FipV and TFEV, respectively, are shown below in Table 4. Partial inhibition was observed at concentrations below the indicated concentrations. In addition, supernatants of infected cells that were treated with GA or GA+M-21-1(met) were titrated with a standard TCID50 assay. A significant reduction of viral replication of more than on log was observed at all concentrations tested that ranged from 100 µM to 500µM for Compound **5** for FipV and TGEV. Virus replication was significantly reduced at a Glycyrrhizin concentration of 500µM for both viruses FipV and TGEV.

**Table 4**

| | | | |
|---|---|---|---|
| Gycyrrhizin Derivative | EC⁵⁰µM, FipV | EC⁵⁰µM, TGEV | Tox. CRFK cells |
| Glycyrrhizin Monoammonium Salt | <500µM | <500µM | >1mM |
| Compound 5 | <100µM | <100µM | >1mM |

### CONCLUSION

Glycyrrhizin Monoammonium Salt and the Glycyrrhizin derivative Compound 5 not only protect cells from virus induced cell death in a dose dependent manner but also inhibit the viral replication of FipV and TGEV in CRFK cells. Thus, Glycyrrhizin Monoammonium Salt and the Glycyrrhizin derivative Compound 5 are useful for the production of veterinary medicines targeting disease caused by said coronaviruses.

### Equivalents

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended claims.

A number of references have been cited, the entire disclosures of which have been incorporated herein in their entireties.

## Claims

1. Use of Glycyrrhizin or a derivative thereof for the preparation of a medicament for preventing or treating a togavirus infection or ameliorating one or more symptoms thereof.

2. The use of claim 1, **characterized in that** the derivative of Glycyrrhizin is a compound of Formula (I): 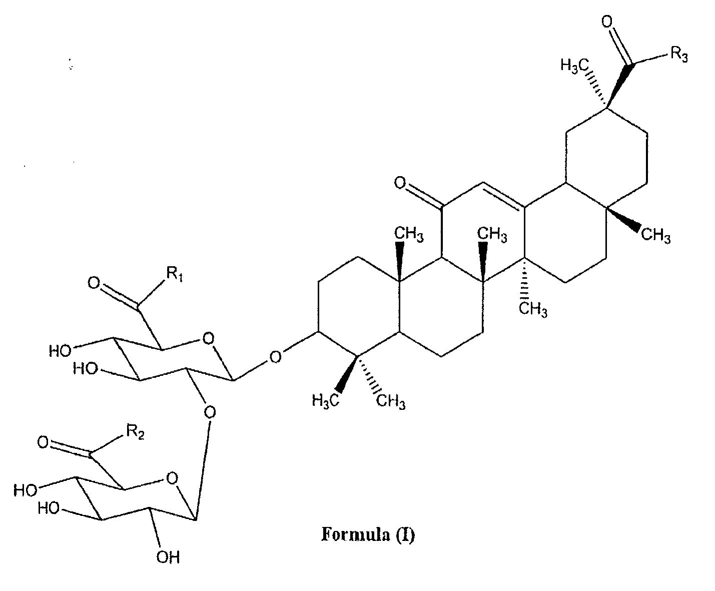 wherein R₁, R₂, and R₃ are independently: -OH; 5-, 6-, or 7-membered heterocycle; -Glycine-Leucine; -N(H)R₄ is -5-,6-, or 7-membered heterocycle.

3. The use of claim 1 or 2, **characterized in that** R₁, R₂, and R₃ each is:

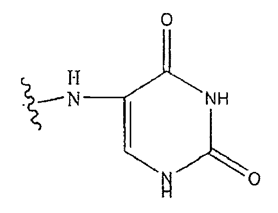

4. The use of claim 1, **characterized in that** the derivative of Glycyrrhizin is a compound of Formula (I): 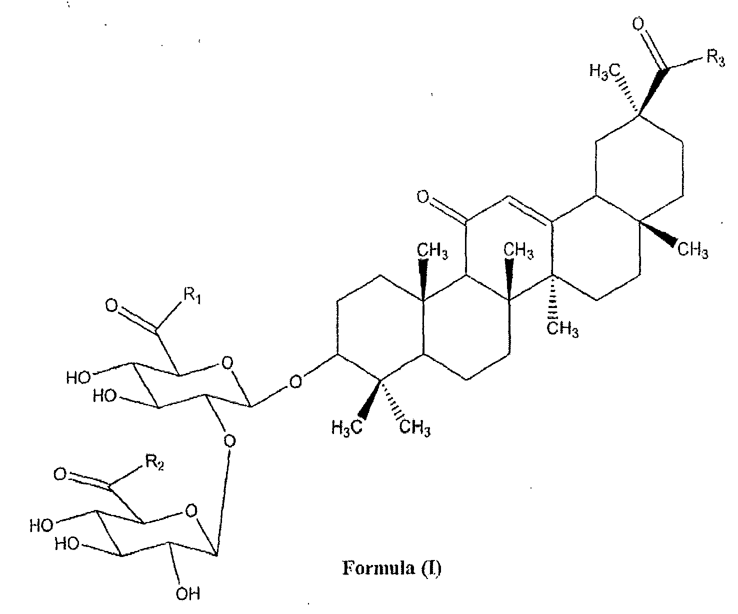 or a pharmaceutically acceptable salt thereof, wherein R₁, R₂, and R₃ are -N(H)R₄, wherein R₄ is -5-, 6-, or 7-membered heterocycle (substituted or unsubstituted), with the proviso that R₄ is not thiazole, uracil or

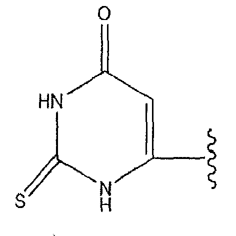

5. The use of claim 1, **characterized in that** the derivative of Glycyrrhizin is a compound of Formula (I): 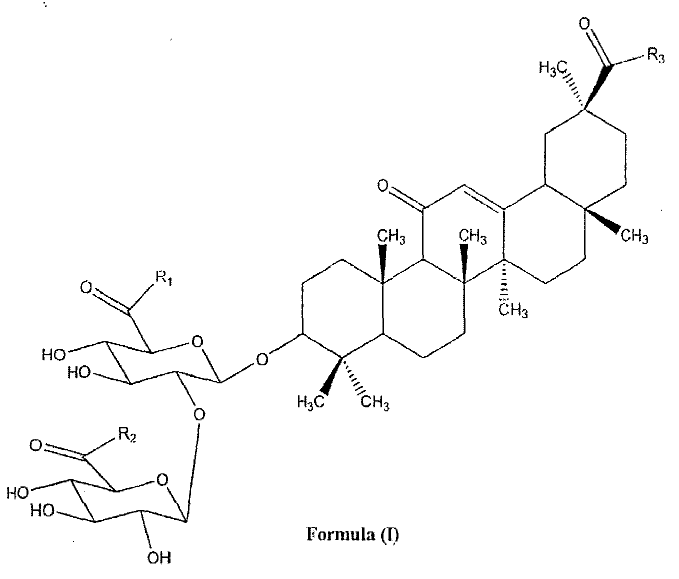 or a pharmaceutically acceptable salt thereof, wherein one of R₁ and R₂ is: 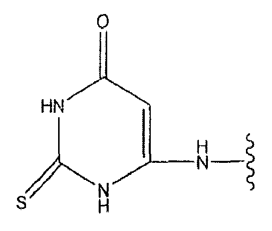 and R₃ and the other of R₁ and R₂ are independently -OH; -OCH₃; -NH-NH₂; -NHCH (COOH)CH₂SCH₂C₆Hs; 5-, 6-, or 7-membered heterocycle (substituted or unsubstituted); an amino acid; a peptide; -N(H)R₄, wherein R₄ is -5-, 6-, or 7-membered heterocycle (substituted or unsubstituted).

6. The use of claim 1, **characterized in that** the derivative of Glycyrrhizin is a compound of Formula (I): 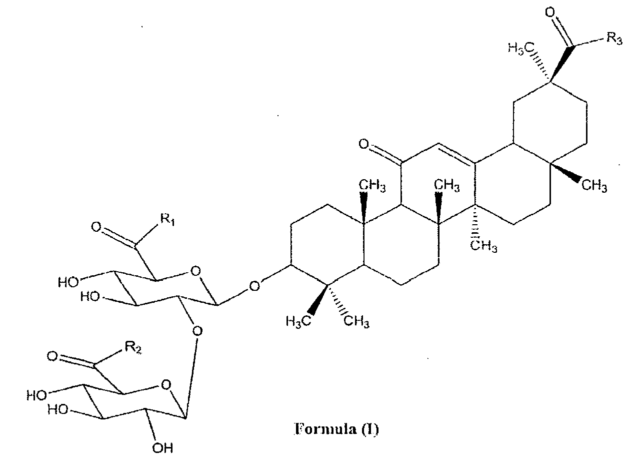 or a pharmaceutically acceptable salt thereof, wherein one of R₁, R₂, and R₃ is an amino acid or a peptide and the other two of R₁, R₂, and R₃ are independently -OH; -OCH₃; -NH-NH₂; -NHCH (COOH)CH₂SCH₂C₆H₅; 5-, 6-, or 7-membered heterocycle (substituted or unsubstituted); -N(H)R₄, wherein R₄ is -5-, 6-, or 7-membered heterocycle (substituted or unsubstituted).

7. The use of claim 1, **characterized in that** the derivative of Glycyrrhizin is a compound of Formula (I): 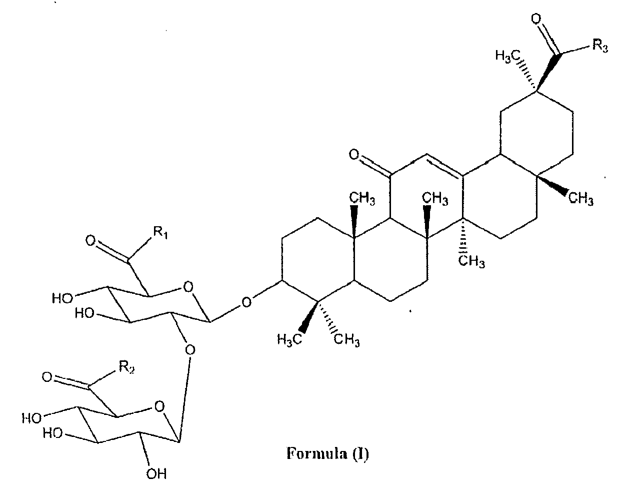 or a pharmaceutically acceptable salt thereof, wherein R₁, R₂, and R₃ are:

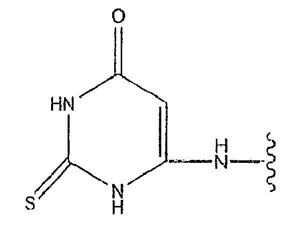

8. The use of claim 1, **characterized in that** the derivative of Glycyrrhizin is a compound of Formula (I): 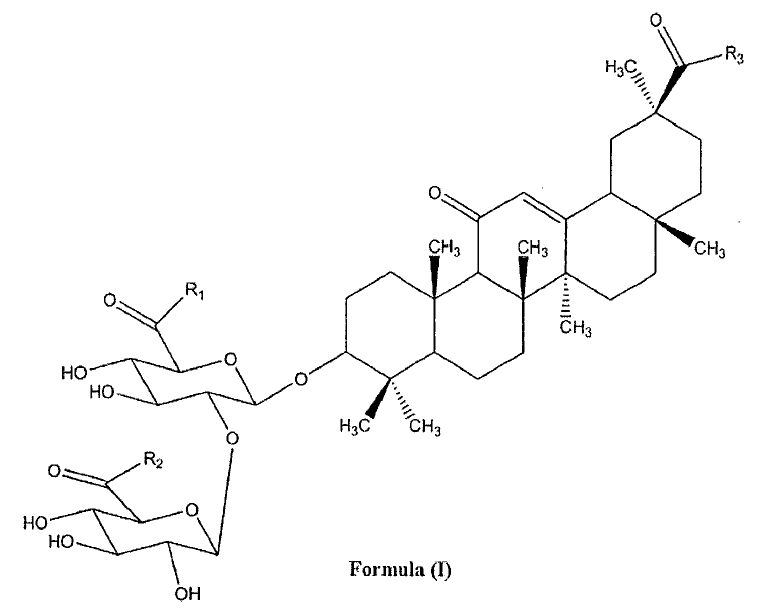 or a pharmaceutically acceptable salt thereof, wherein R₁, R₂, and R₃ are independently a 5-, 6-, or 7-membered heterocycle (substituted or unsubstituted), with the proviso that R₁, R₂, and R₃ are not all proline.

9. The use of claim 1, **characterized in that** the Glycyrrhizin derivative is 18β-Glycyrrhizinic acid.

10. The use of any one of claims 1 to 9, **characterized in that** Glycyrrhizin or the derivative thereof is purified.

11. The use of any one of claims 1 to 10, **characterized in that** the medicament is for the treatment of an elderly human, a human infant or an immunocompromised human infected with a togavirus.

12. The use of any one of claims 1 to 11, **characterized in that** the medicament comprises a therapeutically effective amount of an antiviral agent other than Glycyrrhizin or a derivative thereof.

13. The use of claim 12, **characterized in that** the antiviral agent other than Glycyrrhizin is Ribavirin.

14. The use of any one of claims 1 to 13, **characterized in that** the medicament is for preventing a togavirus infection in a subject with a prophylactically effective amount of Glycyrrhizin or a derivative thereof.

15. The use of claim 14, **characterized in that** the subject has been exposed to a togavirus infection.

16. The use of any one of claims 1 to 13, **characterized in that** the medicament is for treating a togavirus infection in a subject with a therapeutically effective amount of Glycyrrhizin or a derivative thereof.

17. The use of any one of claims 1 to 16, **characterized in that** the togavirus is a Rubella virus or Semliki forest virus.

18. A method of inhibiting or reducing the multiplication of a togavirus, said method comprising contacting a cell in vitro with an effective amount of Glycyrrhizin or a derivative thereof.

19. A method of inhibiting or reducing the production oftogavirus particles, said method comprising contacting a cell in vitro with an effective amount of Glycyrrhizin or a derivative thereof.

20. The method of claim 18 or 19, **characterized in that** the Glycyrrhizin derivative is defined as in any one of claims 2 to 10.
